# EUROPEAN PATENT SPECIFICATION

(11) **EP 0 625 204 B1**
(45) Date of publication and mention of the grant of the patent: **19.06.2002**
(21) Application number: 93904901.1
(22) Date of filing: 04.02.1993
(51) Int. Cl.: C12N 15/867, C12N 15/51, A61K 39/29

(54) **HEPATITIS THERAPEUTICS**
HEPATISTHERAPEUTIKUM
THERAPEUTIQUE DE L'HEPATITE

(30) Priority: 04.02.1992 US 830417
(43) Date of publication of application: 23.11.1994
(73) Proprietor: CHIRON CORPORATION, Emeryville, California 94608 (US)
(72) Inventor: JOLLY, Douglas, J., La Jolla, CA 92037 (US); CHANG, Stephen, M., W., San Diego, CA 92129 (US); LEE, William, Tsung-Liang, Carlsbad, CA 92009 (US); TOWNSEND, Kay, Encinitas, CA 92024 (US); O'DEA, Joann, LaJolla, CA 92037 (US)
(74) Representative: Goldin, Douglas Michael
(86) International application number: US9301009
(87) International publication number: WO9315207

(56) References cited:
- EP-A- 0 450 931
- EP-A- 0 463 848
- EP-A- 0 472 207
- WO-A-88/00971
- WO-A-88/06185
- WO-A-91/15596
- W. YE ET AL.: "Co-expression of hepatitis B virus antigens by a non-defective adenovirus vaccine vector" ARCHIVES OF VIROLOGY, vol. 118, no. 1-2, 1991, pages 11-27, XP002061277
- S. HARADA ET AL.: "Expression of processed core protein of hepatitis C virus in mammalian cells" JOURNAL OF VIROLOGY, vol. 65, no. 6, June 1991, AMERICAN SOCIETY FOR MICROBIOLOGY US, pages 3015-3021, XP002061280
- J. CHIBA ET AL.: "Serodiagnosis of hepatitis C virus (HCV) infection with an HCV core protein molecularly expressed by a recombinant baculovirus" PROCEEDINGS OF THE NATIONAL ACADEMY OF SCIENCES OF USA, vol. 88, no. 11, 1 June 1991, WASHINGTON US, pages 4641-4645, XP002061281
- K. MURAISO ET AL.: "A structural protein of hepatitis C virus expressed in E. coli facilitates accurate detection of hepatitis C virus" BIOCHEMICAL AND BIOPHYSICAL RESEARCH COMMUNICATIONS, vol. 172, no. 2, 30 October 1990, ORLANDO, FL US, pages 511-516, XP002061282

### SEQUENCE LISTING

(1) GENERAL INFORMATION:
   (i) APPLICANT: Chiron Corporation
   (ii) TITLE 0F INVENTION: HEPATITIS THERAPEUTICS
   (iii) NUMBER OF SEQUENCES: 56
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: 93904901.1
      (B) FILING DATE: 4 February 1993
(2) INFORMATION FOR SEQ ID NO:1:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:1:
   CTCGAGCTCG AGGCACCAGC ACCATGCAAC TTTTT 35
(2) INFORMATION FOR SEQ ID NO:2:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:2:
   CTACTAGATC CCTAGATGCT GGATCTTCC 29
(2) INFORMATION FOR SEQ ID NO:3:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:3:
   GGAAGATCCA GCATCTAGGG ATCTAGTAG 29
(2) INFORMATION FOR SEQ ID NO:4:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:4:
   GGGCGATATC AAGCTTATCG ATACCG 26
(2) INFORMATION FOR SEQ ID N0:5:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:6:
   ATTAACCCTC ACTAAAG 17
(2) INFORMATION FOR SEQ ID NO:7:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:7:
   AATACGACTC ACTATAGGG 19
(2) INFORMATION FOR SEQ ID NO:8:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:8:
   CCTCGAGCTC GAGCTTGGGT GGCTTTGGGG CATG 34
(2) INFORMATION FOR SEQ ID N0:9:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 20 base pairs
      (B) TYPE: nucleic acid
      (C) STRANDEDNESS: single -
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:10:
   GTAGACCGTG CATCATGAGC 20
(2) INFORMATION FOR SEQ ID NO:11:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:11:
   ATAGCGGAAC AGAGAGCAGC 20
(2) INFORMATION FOR SEQ ID NO:12:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:12:
   CTCGAGCTCG AGCCACCATG AGCACAAATC CTAAACCTCA AAGAAAAACC AAACG          55
(2) INFORMATION FOR SEQ ID NO:13:
   (i) SEQUENCE CHARACTERISTICS:
      (A) LENGTH: 62 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:13:
   GCAAGCTTAA GCTTCTATCA AGCGGAAGCT GGGATGGTCA AACAAGACAG CAAAGCTAAG 60
   AG 62
(2) INFORMATION FOR SEQ ID N0:14:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:14:
(2) INFORMATION FOR SEQ ID NO:15:
   (i) SEQUENCE CHARACTERISTICS:
      (A) LENGTH: 62 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:15:
   GCCTCGAGCT CGAGCTATCA AGAGGAAGCT GGGATGGTCA AACAAGACAG CAAAGCTAAG 60
   AG 62
(2) INFORMATION FOR SEQ ID N0:16:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:17:
   CGTGGTGTAT GCGTTGATGG 20
(2) INFORMATION FOR SEQ ID NO:18:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:18:
   CCTCGAGCTC GAGCCACCAT GGGGAAGGAG ATACTTCTAG GACCGGCCGA TAGTTTTGG 59
(2) INFORMATION FOR SEQ ID NO:19:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:19:
   GCAAGCTTAA GCTTCTATCA GCGTTGGCAT GACAGGAAAG GGAGTCCCGG TAACCGCGGC 60
(2) INFORMATION FOR SEQ ID NO:20:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:20:
   ATAAATAGAA GGCCTGATAT G 21
(2) INFORMATION FOR SEQ ID NO:21:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:21:
   GCAAGCTTAC AATGTACAGG ATGCAACTCC TGTCT 35
(2) INFORMATION FOR SEQ ID NO:22:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:22:
   GACTCGAGTT ATCAAGTCAG TGTTGAGATG ATGCT 35
(2) INFORMATION FOR SEQ ID N0:23:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:23:
(2) INFORMATION FOR SEQ ID NO:24:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:24:
   GAGGGCCCTT ATCAAGTCAG TGTTGAGATG ATGCT 35
(2) INFORMATION FOR SEQ ID NO:25:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:25:
   CGAAGCTTAA GCTTGCCATG GGCCACACAC GGAGGCAGGG AACATCACCA TCC 53
(2) INFORMATION FOR SEQ ID NO:26:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:26:
   CCTCGAGCTC GAGCTGTTAT ACAGGGCGTA CACTTTCCCT TCTCAATCTC TC 52
(2) INFORMATION FOR SEQ ID NO:27:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:27:
   CCTCGAGCTC GAGGCCATGG GCCACACACG GAGGCAGGGA ACATCACCAT CC 52
(2) INFORMATION FOR SEQ ID NO:28:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:28:
   CGGGCCCGGG CCCCTGTTAT ACAGGGCGTA CACTTTCCCT TCTCAATCTC TC 52
(2) INFORMATION FOR SEQ ID NO:29:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:29:
   GCAAGCTTAA GCTTGAGGAT GTGGCTGCAG AGCCTGCTGC TCTTGGGCAC TGTGGCCTGC 60
   AGCATCTCTG CA 72
(2) INFORMATION FOR SEQ ID NO:30:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:30:
   TCCTGGATGG CATTCACATG CTCCCAGGGC TGCGTGCTGG GGCTGGGCGA GCGGGCGGGT 60
   GCAGAGATGC TGCAG 75
(2) INFORMATION FOR SEQ ID NO:31:
   (i) SEQUENCE CHARACTERISTICS:
      (A) LENGTH: 61 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:31:
   GAATGCCATC CAGGAGGCCC GGCGTCTCCT GAACCTGAGT AGAGACACTG CTGCTGAGAT 60
   G 61
(2) INFORMATION FOR SEQ ID N0:32:
   (i) SEQUENCE CHARACTERISTICS:
      (A) LENGTH: 96 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:32:
(2) INFORMATION FOR SEQ ID NO:33:
   (i) SEQUENCE CHARACTERISTICS:
      (A) LENGTH: 90 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:33:
   CCTGGAGCTG TACAAGCAGG GCCTGCGGGG CAGCCTCACC AAGCTCAAGG GCCCCTTGAC 60
   CATGATGGCC AGCCACTACA AGCAGCACTG 90
(2) INFORMATION FOR SEQ ID N0:34:
   (i) SEQUENCE CHARACTERISTICS:
      (A) LENGTH: 58 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:34:
(2) INFORMATION FOR SEQ ID N0:35:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:35:
(2) INFORMATION FOR SEQ ID N0:36:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:36:
(2) INFORMATION FOR SEQ ID NO:37:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:37:
   GCTCTAGATC TAGAGTCTCA CTCCTGGACT GGCTCCCAGC AGTCAAAGGG GATGACAAGC 60
   AGAAAGTCC 69
(2) INFORMATION FOR SEQ ID N0:38:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:38:
(2) INFORMATION FOR SEQ ID N0:39:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:39:
(2) INFORMATION FOR SEQ ID NO:40:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:40:
   GGAAAGAAGT CAGAAGGCAA 20
(2) INFORMATION FOR SEQ ID NO:41:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:41:
   GCCTCGAGCT CGAGGAGGAT GTGGCTGCAG AGCCTGCTGC TCTTGGGCAC TGTGGCCTGC 60
   AGCATCTCTG CA 72
(2) INFORMATION FOR SEQ ID NO:42:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:42:
   GCCTCGAGCT CGAGGTCATC CTCAGGCCAT GCAGTGGAAT TCCACTGCCT TGCACCAAGC 60
   TCTGCAGG 68
(2) INFORMATION FOR SEQ ID NO:43:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:43:
   GCATCGATAT CGATGTTCCC CAACTTCCAA TTATGTAGCC CATGAAGTTT AGGGAATAAC 60
   CCC 63
(2) INFORMATION FOR SEQ ID NO:44:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:44:
   GCCTCGAGCT CGAGACCATG CCCCTATCTT ATCAACACTT CCGGAAACTA CTGTTGTTAG 60
   ACGACGGGAC CGAGGCAGG 79
(2) INFORMATION FOR SEQ ID NO:45:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:45:
   GCATCGATAT CGATGGGCAG GATCTGATGG GCGTTCACGG TGGTCGCCAT GCAACGTGCA 60
   GAGGTG 66
(2) INFORMATION FOR SEQ ID NO:46:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:46:
   GCCTCGAGCT CGAGACCATG TCCCGTCGGC GCTGAATCCC GCGGACGACC CCTCTCGGGG 60
   CCGCTTGGGA C 71
(2) INFORMATION FOR SEQ ID NO:47:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:47:
   GCATCGATAT CGATGGTCGG TCGTTGACAT TGCTGGGAGT CCAAGAGTCC TCTTATGTAA 60
   GACC 64
(2) INFORMATION FOR SEQ ID NO:48:
   (i) SEQUENCE CHARACTERISTICS:
      (A) LENGTH: 74 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:48:
   GCCTCGAGCT CGAGACCATG ATTAGGCAGA GGTGAAAAAG TTGCATGGTG CTGGTGCGCA 60
   GACCAATTTA TGCC 74
(2) INFORMATION FOR SEQ ID NO:49:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:49:
   GCATCGATAT CGATGCTGAC GCAACCCCCA CTGGCTGGGG CTTAGCCATA GGCCATCAGC 60
   GCATGCG 67
(2) INFORMATION FOR SEQ ID NO:50:
   (i) SEQUENCE CHARACTERISTICS:
      (A) LENGTH: 655 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (v) FRAGMENT TYPE: N-terminal
   (xi) SEQUENCE DESCRIPTION: SEQ ID NO:50:
   CACCAGCAAC ATGCAACTTT TTCACCTCTG CCTAATCATC TCTTGTACAT GTCCCACTGT    60
   TCAAGCCTCC AAGCTGTGCC TTGGGTGGCT TTGGGGCATG GACATTGACC CTTATAAAGA   120
   ATTTGGAGCT ACTGTGGAGT TACTCTCGTT TTTGCCTTCT GACTTCTTTC CTTCCGTCAG   180
   AGATCTCCTA GACACCGCCT CAGCTCTGTA TCGGGAAGCC TTAGAGTCTC CTGAGCATTG   240
   CTCACCTCAC CACACCGCAC TCAGGCAAGC CATTCTCTGC TGGGGGGAAT TGATGACTCT   300
   AGCTACCTGG GTGGGTAATA ATTTGGAAGA TCCAGCATCT AGGGATCTAG TAGTCAATTA   360
   TGTTAATACT AACATGGGTT TAAAAATTAG GCAACTATTG TGGTTTCATA TATCTTGCCT   420
   TACTTTTGGA AGAGAGACTG TACTTGAATA TTTGGTATCT TTCGGAGTGT GGATTCGCAC   480
   TCCTCCAGCC TATAGACCAC CAAATGCCCC TATCTTATCA ACACTTCCGG AAACTACTGT   540
   TGTTAGACGA CGGGACCGAG GCAGGTCCCC TAGAAGAAGA ACTCCCTCGC CTCGCAGACG   600
   CAGATCTCCA TCGCCGCGTC GCAGAAGATC TCAATCTCGG GAATCTCAAT GTTAG        655

## Description

### Technical Field

The present invention relates generally to methods for treating hepatitis, as well as hepatitis-associated carcinomas.

### Background of the Invention

Hepatitis is a systemic disease which predominantly affects the liver. The disease is typified by the initial onset of symptoms such as anorexia, nausea, vomiting, fatigue, malaise, arthralgias, myalgias, and headaches. followed by the onset of jaundice. The disease may also be characterized by increased serum levels of the aminotransferases AST and ALT. Quantification of these enzymes in serum indicates the extent of liver damage.

There are five general categories of viral agents which have been associated with hepatitis: the hepatitis A virus (HAV); the hepatitis B virus (HBV); two types of non-A, non-B (NANB) agents, one blood-borne (hepatitis C) and the other enterically transmitted (hepatitis E); and the HBV-associated delta agent (hepatitis D).

There are two general clinical categories of hepatitis, acute hepatitis and chronic hepatitis. Symptoms for acute hepatitis range from asymptomatic and non-apparent to fatal infections. The disease may be subclinical and persistent, or rapidly progress to chronic liver disease with cirrhosis, and in some cases, to hepatocellular carcinoma. Acute hepatitis B infection in adult Caucasians in the United States progresses to chronic hepatitis B in about 5% to 10% of the cases. In the remainder of the cases, approximately 65% are asymptomatic. In the Far East, infection is usually perinatal, and 50% to 90% progress to the chronic state. It is likely that the different rates of progression are linked to the age at infection rather than genetic differences in the hosts. In the United States, about 0.2% of the population is chronically infected, with higher percentages in high-risk groups such as physicians, drug addicts and renal dialysis patients. In countries such as Taiwan, Hong Kong and Singapore, the level in the population with hepatitis infection may be as high as 10%.

In the United States, about 20% of patients with chronic hepatitis die of liver failure, and a further 5% develop hepatitis B-associated carcinoma. In the Far East, a large percentage of the population is infected with HBV, and after a long chronic infection (20 to 40 years), approximately 25% of these will develop hepatocellular carcinoma.

Until recently, no therapy has proven effective for treatment of acute or chronic hepatitis B, and patients infected with hepatitis must generally allow the disease run its course. Most anti-viral drugs, such as acyclovir, as well as attempts to bolster the immune system through the use of corticosteroids have proven ineffective (Alter, "Viral hepatitis and liver disease," Zuckerman (ed.), New York: Alan R. Liss, pp. 537-42, 1988). Some anti-viral activity has been observed with adenosine arabinoside (Jacyna et al., *British Med. Bull. 46*:368-382, 1990), although toxic side effects which are associated with this drug render such treatment unacceptable.

One treatment that has provided some benefit for chronic hepatitis B and C infections is the use of recombinant alpha interferon (Davis et al., *New Eng. J. Med. 321*(22):1501-1506, 1989; Perrillo et al., *New Eng. J. Med*. *323*:295-301, 1990). However, for patients with hepatitis B infections only about 35% of infectees responded to such treatment, and in perinatal infectees only about 10% responded to treatment. In addition, a further difficulty with alpha interferon therapy is that the composition frequently has toxic side effects which require reduced dosages for sensitive patients.

Therefore, therapeutics that could serve to augment natural host defenses against hepatitis, or against tumor induction and progression, with reduced cytotoxicity, or that allows treatment of interferon non-responsive individuals would be very beneficial. The present invention provides such therapeutic agents, and further provides other related advantages.

### Summary of the Invention

Briefly stated, the present invention is directed toward recombinant retroviruses for use in methods of treating hepatitis B. Within one aspect of the present invention, a recombinant retrovirus is provided which carries a vector construct which directs the expression of at least one immunogenic portion of the hepatitis B antigen HBcAg for use in treating hepatitis B infections. The invention also provides a recombinant retrovirus which carries a vector construct which directs the expression of an immunogenic portion of hepatitis B antigen and an immunomodulatory cofactor for use in treating hepatitis B infections.

Within a related aspect of the present invention, a recombinant retrovirus which carries a vector construct is provided which directs the co-expression of at least one immunogenic portion of the hepatitis B antigen HBcAg and a immunomodulatory cofactor. Also provided are pharmaceutical compositions comprising the recombinant retroviruses of the invention in combination with a pharmaceutically acceptable carrier or diluent.

Within another aspect of the invention, a kit is provided for treating hepatitis B infections which kit comprises a recombinant retrovirus of the invention and an immunomodulatory cofactor.

These and other aspects of the present invention will become evident upon reference to the following detailed description and attached drawings.

### Brief Description of the Drawings

Figure 1 is a schematic illustration which outlines the recovery of Hepatitis B e sequence from ATCC 45020.

Figure 2 is a diagrammatic representation of the nucleotide sequence of HBV (*adw*) precore/core (SEQ ID. NO. 50) and the corrected sequences.

Figure 3 is a schematic representation of the correction of the deletion in HBe-c sequence from pAM6 (ATCC 45020).

Figure 4 is a DNA sequencing gel showing the corrected nucleotide sequences from SK⁺HBe-c.

Figure 5 is a chart showing expression of HBVe protein from the following retrovirally transduced murine cell lines BC10ME, Bl/6, L-M(TK⁻), EL4, and retrovirally transduced EBV-transformed human B-cell line, JY-LCL, as determined by ELISA.

Figure 6 is a Western blot showing expression of p17 kD HBV e protein secreted by retrovirally transduced BC10ME and Bl/6 cells and p22 and p23 kD precore intermediate proteins in cell lysates from retrovirally transduced BC10ME and B1/6 cells.

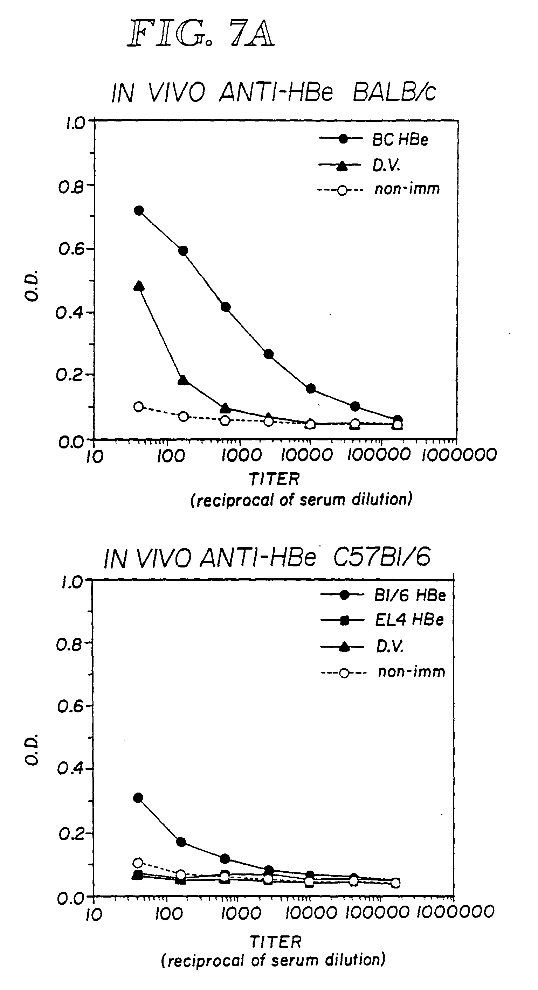

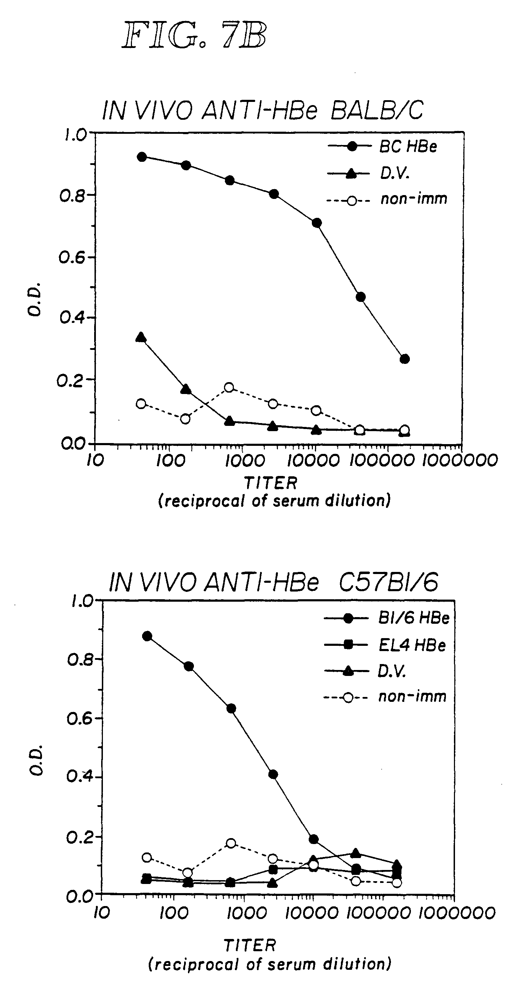

Figure 7 is a graph showing induction of antibody responses against HBVe antigen in Balb/C and C57Bl/6 mice injected with syngeneic cells expressing the antigen or by direct injection with the retroviral vector encoding HBVe antigen.

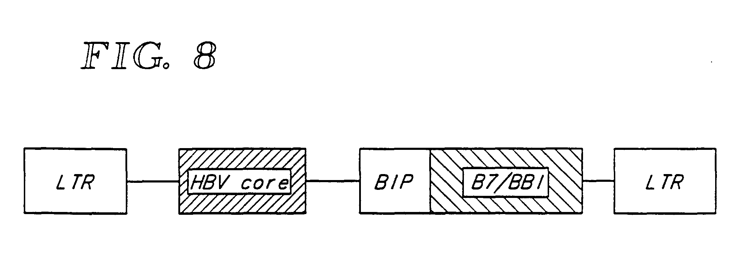

Figure 8 is a diagrammatic representation of vector construct KT-HBV core/B7/BB1 which expresses both HBV core and B7/BB1 proteins.

### Detailed Description of the Invention

Prior to setting forth the invention, it may be helpful to an understanding thereof to first define certain terms that will be used hereinafter. All references which have been cited below are hereby incorporated by reference in their entirety.

"Immunogenic portion" as utilized within the present invention, refers to a portion of the respective antigen which is capable, under the appropriate conditions, of causing an immune response (*i.e.*, cell-mediated or humoral). "Portions" may be of variable size, but are preferably at least 9 amino acids long, and may include the entire antigen. Representative assays which may be utilized to determine immunogenicity (*e.g.*, cell-mediated immune response), are described in more detail below, as well as in Example 12. Cell mediated immune responses may be mediated through Major Histocompatability Complex ("MHC") class I presentation, MHC Class II presentation, or both.

"Vector construct" refers to an assembly which is capable of directing the expression of the sequence(s) or gene(s) of interest. The vector construct must include promoter elements and preferably includes a signal that directs poly-adenylation. In addition, the vector construct must include a sequence which, when transcribed, is operably linked to the sequence(s) or gene(s) of interest and acts as a translation initiation sequence. Preferably, the vector construct also includes a selectable marker such as Neo, SV₂ Neo, TK, hygromycin, phleomycin, histidinol, or DHFR, as well as one or more restriction sites and a translation termination sequence. In addition, if the vector construct is placed into a retrovirus, the vector construct must include a packaging signal and long terminal repeats (LTRs) appropriate to the retrovirus used (if these are not already present).

"Immunomodulatory cofactor" refers to factors which, when manufactured by one or more of the cells involved in an immune response, or, which when added exogenously to the cells, causes the immune response to be different in quality or potency from that which would have occurred in the absence of the cofactor. The quality or potency of a response may be measured by a variety of assays known to one of skill in the art including, for example, *in vitro* assays which measure cellular proliferation (*e.g.,* ³H thymidine uptake), and *in vitro* cytotoxic assays (*e.g.*, which measure ⁵¹Cr release) (*see,* Warner et al., *AIDS Res. and Human Retroviruses 7*:645-655, 1991). Immunomodulatory cofactors may be active both *in vivo* and *ex vivo.* Representative examples of such cofactors include cytokines, such as interleukins 2, 4, and 6 (among others), alpha interferons, beta interferons, gamma interferons, GM-CSF, G-CSF, and tumor necrosis factors (TNFs). Other immunomodulatory cofactors include, for example, CD3, ICAM-1, ICAM-2, LFA-1, LFA-3, MHC class I molecules, MHC class II molecules, B7/BB1, β₂-microglobulin, chaperones, or analogs thereof.

As noted above, the present invention is directed towards methods and compositions for treating hepatitis B infections. Briefly, the ability to recognize and defend against foreign pathogens is central to the function of the immune system. This system, through immune recognition, is capable of distinguishing "self' from "nonself" (foreign), which is essential to ensure that defensive -mechanisms are directed towards invading entities rather than against host tissues. The methods which are described in greater detail below provide an effective means of inducing potent class I-restricted protective and therapeutic CTL responses, as well as humoral responses.

As noted above, within one aspect of the present invention, a method for treating hepatitis B infections in warm-blooded animals is provided, comprising the step of administering a vector construct to a warm-blooded animal which directs the expression of at least one immunogenic portion of a hepatitis B antigen, such that an immune response is generated.

Briefly, the hepatitis B genome is comprised of circular DNA of about 3.2 kilobases in length, and has been well characterized (Tiollais et al., *Science 213*:406-411, 1981; Tiollais et al., *Nature 317*:489-495, 1985; and Ganem and Varmus, *Ann. Rev. Biochem. 56*:651-693, 1987). The hepatitis B virus presents several different antigens, including among others, three HB "S" antigens (HBsAgs), an HBc antigen (HBcAg), an HBe antigen (HBeAg), and an HBx antigen (HBxAg) (*see* Blum et al., "The Molecular Biology of Hepatitis B Virus," *TIG 5*(5):154-158, 1989). Briefly, the HBeAg results from proteolytic cleavage of P22 precore intermediate and is secreted from the cell. HBeAg is found in serum as a 17 kD protein. The HBcAg is a protein of 183 amino acids, and the HBxAg is a protein of 145 to 154 amino acids, depending on subtype.

The HBsAgs (designated "large," "middle," and "small") are encoded by three regions of the hepatitis B genome: S, pre-S2 and pre-S1. The large protein, which has a length varying from 389 to 400 amino acids, is encoded by pre-S1, pre-S2, and S regions, and is found in glycosylated and non-glycosylated forms. The middle protein is 281 amino acids long and is encoded by the pre-S2 and S regions. The small protein is 226 amino acids long and is encoded by the S region. It exists in two forms, glycosylated (GP 27^{S}) and non-glycosylated (P24^{S}). If each of these regions are expressed separately, the pre-S1 region will code for a protein of approximately 119 amino acids, the pre-S2 region will code for a protein of approximately 55 amino acids, and the S region will code for a protein of approximately 226 amino acids.

As will be evident to one of ordinary skill in the art, various immunogenic portions of the above described S antigens may be combined in order to present an immune response when administered by one of the vector constructs described herein. In addition, due to the large immunological variability that is found in different geographic regions for the S open reading frame of HBV, particular combinations of antigens may be preferred for administration in particular geographic regions. Briefly, epitopes that are found in all human hepatitis B virus S samples are defined as determinant "α". Mutually exclusive subtype determinants however have also been identified by two-dimensional double immunodiffusion (Ouchterlony, *Progr. Allergy 5*:1, *1958*). These determinants have been designated *"d"* or "*y*" and "*w*" or "*r*" (LeBouvier, *J. Infect. 123*:671, 1971; Bancroft et al., *J. Immunol. 109*:842, 1972; Courouce et al., *Bibl. Haematol. 42*:1-158, 1976). The immunological variability, is due to single nucleotide substitutions in two areas of the hepatitis B virus S open reading frame: (1) exchange of lysine-122 to arginine in the hepatitis B virus S open reading frame causes a subtype shift from *d* to *y*, and (2) exchange of arginine-160 to lysine causes the shift from subtype *r* to *w.* In black Africa, subtype *ayw* is predominant, whereas in the U.S. and northern Europe the subtype *adw*₂ is more abundant (*Molecular Biology of the Hepatitis B Virus*, McLachlan (ed.), CRC Press, 1991). As will be evident to one of ordinary skill in the art, it is generally preferred to construct a vector for administration which is appropriate to the particular hepatitis B virus subtype which is prevalent in the geographical region of administration. Subtypes of a particular region may be determined by two-dimensional double immunodiffusion or, preferably, by sequencing the S open reading frame of HBV virus isolated from individuals within that region.

Also presented by HBV are pol ("HBV pol"), ORF 5, and ORF 6 antigens. Briefly, the polymerase open reading frame of HBV encodes reverse transcriptase activity found in virions and core-like particles in infected liver. The polymerase protein consists of at least two domains: the amino terminal domain encodes the protein that primes reverse transcription, and the carboxyl terminal domain which encodes reverse transcriptase and RNAse H activity. Immunogenic portions of HBV pol may be determined utilizing methods described herein (*e.g.*, below and in Examples 11Aii and 12), expressed utilizing vector constructs described below, and administered in order to generate an immune response within a warm-blooded animal. Similarly, other HBV antigens such as ORF 5 and ORF 6, (Miller et al., *Hepatology 9*:322-327, 1989), may be expressed utilizing vector constructs as described herein. Representative examples of vector constructs utilizing ORF 5 and ORF 6 are set forth below in Examples 2J, 2K and 4E, 4F.

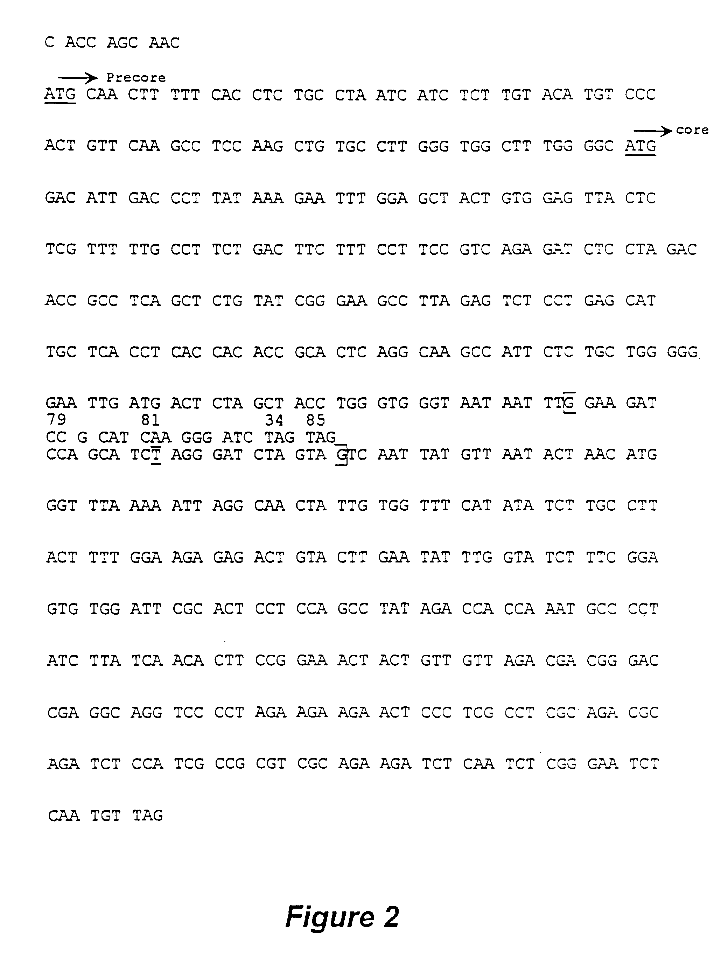

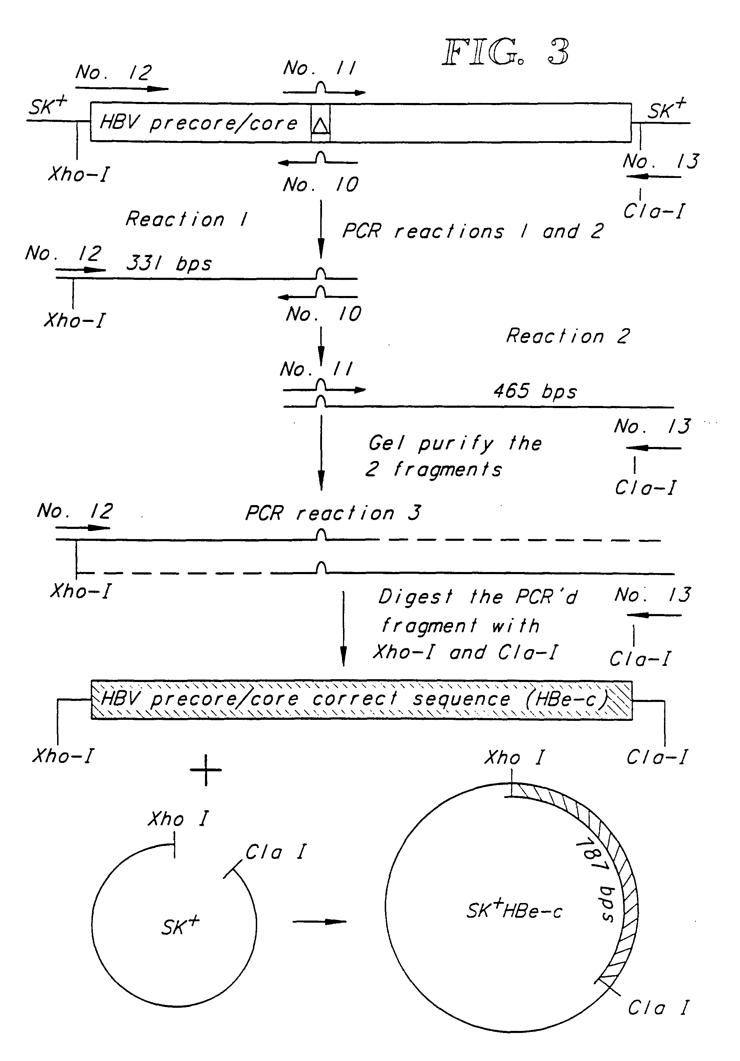

Molecularly cloned genomes which encode the hepatitis B virus may be obtained from a variety of sources including, for example, the American Type Culture Collection (ATCC, Rockville, Maryland). For example, ATCC No. 45020 contains the total genomic DNA of hepatitis B (extracted from purified Dane particles) (*see* Figure 3 of Blum et al., *TIG 5*(5):154-158, 1989) in the Bam HI site of pBR322 (Moriarty et al., *Proc*. *Natl. Acad. Sci. USA 78*:2606-2610, 1981). (Note that, as described in Example 2A and as shown in Figure 2, correctable errors occur in the sequence of ATCC No. 45020.)

As noted above, at least one immunogenic portion of a hepatitis B antigen is incorporated into a vector construct. The immunogenic portion(s) which are incorporated into the vector construct may be of varying length, although it is generally preferred that the portions be at least 9 amino acids long, and may include the entire antigen. Immunogenicity of a particular sequence is often difficult to predict, although T cell epitopes may be predicted utilizing computer algorithms such as TSITES (MedImmune, Maryland), in order to scan coding regions for potential T-helper sites and CTL sites. From this analysis, peptides are synthesized and used as targets in an in *vitro* cytotoxic assay, such as that described in Example 12. Other assays, however, may also be utilized, including, for example, ELISA which detects the presence of antibodies against the newly introduced vector, as well as assays which test for T helper cells, such as gamma-interferon assays, IL-2 production assays, and proliferation assays. A particularly preferred assay is described in more detail below in Example 11B.

Immunogenic portions may also be selected by other methods. For example, the HLA A2.1 transgenic mouse has been shown to be useful as a model for human T-cell recognition of viral antigens. Briefly, in the influenza and hepatitis B viral systems, the murine T-cell receptor repertoire recognizes the same antigenic determinants recognized by human T-cells. In both systems, the CTL response generated in the HLA A2.1 transgenic mouse is directed toward virtually the same epitope as those recognized by human CTLs of the HLA A2.1 haplotype (Vitiello et al., *J. Exp. Med. 173*:1007-1015, 1991; Vitiello et al., *Abstract of Molecular Biology of Hepatitis B Virus Symposia*, 1992).

Particularly preferred immunogenic portions for incorporation into vector constructs include HBeAg, HBcAg, and HBsAgs as described in greater detail below in the Examples.

Additional immunogenic portions of the hepatitis B virus may be obtained by truncating the coding sequence at various locations including, for example, the following sites: Bst UI, Ssp I, Ppu M1, and Msp I (Valenzuela et al., *Nature 280*:815-19, 1979; Valenzuela et al., A*nimal Virus Genetics: ICN*/*UCLA Symp. Mol. Cell Biol.*, 1980, B. N. Fields and R. Jaenisch (eds.), pp. 57-70, New York: Academic).

As noted above, more than one immunogenic portion may be incorporated into the vector construct. For example, a vector construct may express (either separately or as one construct) all or portions of HBcAg, HBeAg, HBsAgs, HBxAg as well as immunogenic portions of HCV antigens as discussed below. In addition, the vector construct may also co-express an immunomodulatory cofactor, such as alpha interferon (Finter et al., *Drugs 42*(5):749-765, 1991; U.S. Patent No. 4,892,743; U.S. Patent No. 4,966,843; WO 85/02862; Nagata et al., Nature 284:316-320, 1980; Familletti et al., *Methods in Enz. 78*:387-394, 1981; Twu et al., *Proc. Natl. Acad. Sci. USA 86*:2046-2050, 1989; Faktor et al., *Oncogene 5*:867-872, 1990), beta interferon (Seif et al., *J. Virol. 65*:664-671, 1991), gamma interferons (Radford et al., *The American Society of Hepatology* 20082015, 1991; Watanabe et al., *PNAS 86*:9456-9460, 1989; Gansbacher et al., *Cancer Research 50*:7820-7825, 1990; Maio et al., *Can. Immunol. Immunother. 30*:34-42, 1989; U.S. Patent No. 4,762,791; U.S. Patent No. 4,727,138), G-CSF (U.S. Patent Nos. 4,999,291 and 4,810,643), GM-CSF (WO 85/04188), TNFs (Jayaraman et al., *J. Immunology 144*:942-951, 1990), Interleukin- 2 (IL-2) (Karupiah et al., *J. Immunology 144*:290-298, 1990; Weber et al., *J. Exp. Med. 166*:1716-1733, 1987; Gansbacher et al., *J. Exp. Med. 172*:1217-1224, 1990; U.S. Patent No. 4,738,927), IL-4 (Tepper et al., *Cell 57*:503-512, 1989; Golumbek et al., *Science 254*:713-716, 1991; U.S. Patent No. 5,017,691), IL-6 (Brakenhof et al., *J. Immunol. 139*:4116-4121, 1987; WO 90/06370), ICAM-1 (Altman et al., *Nature 338*:512-514, 1989), ICAM-2, LFA-1, LFA-3, MHC class I molecules, MHC class II molecules, β₂-microglobulin, chaperones, CD3, B7/BB1, MHC linked transporter proteins or analogs thereof.

The choice of which immunomodulatory cofactor to include within a vector construct may be based upon known therapeutic effects of the cofactor, or, experimentally determined. For example, in chronic hepatitis B infections alpha interferon has been found to be efficacious in compensating a patient's immunological deficit, and thereby assisting recovery from the disease. Alternatively, a suitable immunomodulatory cofactor may be experimentally determined. Briefly, blood samples are first taken from patients with a hepatic disease. Peripheral blood lymphocytes (PBLs) are restimulated *in vitro* with autologous or HLA matched cells (*e.g.*, EBV transformed cells), and transduced with a vector construct which directs the expression of an immunogenic portion of a hepatitis antigen and the immunomodulatory cofactor. Stimulated PBLs are used as effectors in a CTL assay with the HLA matched transduced cells as targets. An increase in CTL response over that seen in the same assay performed using HLA matched stimulator and target cells transduced with a vector encoding the antigen alone, indicates a useful immunomodulatory cofactor.

Molecules which encode the above-described immunomodulatory cofactors may be obtained from a variety of sources. For example, plasmids which contain these sequences may be obtained from a depository such as the American Type Culture Collection (ATCC, Rockville, Maryland), or from commercial sources such as British Bio-technology Limited (Cowley, Oxford England). Representative examples include BBG 12 (containing the GM-CSF gene coding for the mature protein of 127 amino acids), BBG 6 (which contains sequences encoding gamma interferon), ATCC No. 39656 (which contains sequences encoding TNF), ATCC No. 20663 (which contains sequences encoding alpha interferon), ATCC Nos. 31902, 31902 and 39517 (which contains sequences encoding beta interferon), ATCC Nos. 39405, 39452, 39516, 39626 and 39673 (which contains sequences encoding Interleukin-2), ATCC No. 57592 (which contains sequences encoding Interleukin-4), and ATCC 67153 (which contains sequences encoding Interleukin-6).

In a similar manner, sequences which encode immunomodulatory cofactors may be readily obtained from cells which express or contain sequences which encode these cofactors. Briefly, within one embodiment, primers are prepared on either side of the desired sequence, which is subsequently amplified by PCR *(see* U.S. Patent Nos. 4,683,202, 4,683,195 and 4,800,159) (*see* also *PCR Technology: Principles and Applications for DNA Amplification*, Erlich (ed.), Stockton Press, 1989). In particular, a double-stranded DNA is denatured by heating in the presence of heat stable Taq polymerase, sequence specific DNA primers, ATP, CTP, GTP and TTP. Double-stranded DNA is produced when synthesis is complete. This cycle may be repeated many times, resulting in a factorial amplification of the desired DNA.

Sequences which encode immunomodulatory cofactors may also be synthesized, for example, on an Applied Biosystems Inc. DNA synthesizer (*e.g.*, ABI DNA synthesizer model 392 (Foster City, California)). Such sequences may also be linked together through complementary ends, followed by PCR amplification (Vent polymerase, New England Biomedical, Beverly, Massachusetts) to form long double-stranded DNA molecules (Foguet et al., *Biotechniques 13*:674-675, 1992).

Once an immunogenic portion(s) (and, if desired, an immunomodulatory cofactor) have been selected, genes which encode these proteins are placed into a vector construct which directs their expression. In general, such vectors encode only these genes, and no selectable marker. Vectors encoding and leading to expression of a specific antigen and immunomodulatory cofactor may be readily constructed by those skilled in the art. In particular, construction of vector constructs as well as administration of retroviral constructs by direct injection is described in greater detail in an application entitled "Recombinant Retroviruses" (U.S.S.N. 07/586,603, filed September 21, 1990). These vector constructs may be used to generate transduction competent retroviral vector particles by introducing them into appropriate packaging cell lines (*see* U.S.S.N. 07/800,921) in order to generate producer cell lines for the production of retroviral vector particle which are replication incompetent.

Various assays may be utilized in order to detect the presence of any replication competent infectious retroviruses. One preferred assay is the extended S⁺L⁻ assay described in Example 7.

Within a particularly preferred embodiment, vector constructs may be constructed to include a promoter such as SV40 *(see* Kriegler et al., *Cell 38*:483, 1984), cytomegalovirus ("CMV") (*see* Boshart et al., *Cell 41*:521-530, 1991), or an internal ribosomal binding site ("IRBS"). Briefly, with respect to IRBS, the five prime untranslated region of the immunoglobulin heavy chain binding protein has been shown to support the internal engagement of a bicistronic message (*see* Jacejak and Sarnow, *Nature 353*:90-94, 1991). This sequence is small (300 bp), and may readily be incorporated into a retroviral vector in order to express multiple genes from a multi-cistronic message whose cistrons begin with this sequence. A representative vector construct utilizing IRBS is set forth in more detail below in Examples 5C and 5D.

Once a vector construct has been prepared, it may be administered to a warm-blooded animal in order to treat a hepatitis B infection. Methods for administering a vector construct via a retroviral vector (such as by direct injection of the retroviral construct) are described in greater detail in an application entitled "Recombinant Retroviruses" (U.S.S.N. 07/586,603). Such methods include, for example, transfection by methods utilizing various physical methods, such as lipofection (Felgner et al., *Proc. Natl. Acad. Sci. USA 84*:7413-7417, 1989), direct DNA injection (Acsadi et al., *Nature 352*:815-818, 1991); microprojectile bombardment (Williams et al., *PNAS 88*:2726-2730, 1991); liposomes (Wang et al., *PNAS 84*:7851-7855, 1987); CaPO₄ (Dubensky et al., *PNAS 81*:7529-7533, 1984); or DNA ligand (Wu et al., *J*. *Biol. Chem. 264*:16985-16987, 1989). In addition, the vector construct, or nucleic acids which encode the relevant immunogenic portion, may be administered to a patient directly, for example by transfection methods such as lipofection, direct DNA injection, microprojectile bombardment, liposomes, CaPO₄, or DNA ligand. Compositions and methods suitable for administering immunogenic proteins themselves, vector constructs, viral vectors, or viral vectors along with immunomodulatory cofactors, are discussed in more detail below.

As noted above, once an immunogenic portion has been selected, it is generally preferable to ensure that it is non-tumorigenic. This may be accomplished by a variety of methods, including for example by truncation, point mutation, addition of premature stop codons, or phosphorylation site alteration. The polyprotein antigen or modified version thereof may also be tested for tumorigenicity utilizing the above-described methods, or by the methods described in Example 13.

Immunogenic portion(s) (as well as immunomodulatory cofactors, if desired) may then be inserted into a vector construct, and carried by a recombinant virus as described above. Compositions and methods suitable for administering the immunogenic proteins themselves, vector constructs, viral vectors, or viral vectors along with immunomodulatory cofactors, are discussed in more detail below.

Within another aspect of the present invention, vector constructs may be prepared which direct the co-expression of several of the above described immunogenic portions (as well as immunomodulatory co-factors, if desired). For example, within one embodiment vector constructs may be prepared which direct the co-expression of both an immunogenic portion of the hepatitis B antigen. Such constructs may be administered as described above and below, in order to prevent or treat acute and chronic hepatitis B infections.

As noted above, various methods may be utilized to administer vector constructs of the present invention, or nucleic acids which encode the immunogenic portion(s) discussed above, to warm-blooded animals such as humans, directly (Curiel et al., *Human Gene and Therapy 3*:147-154, 1992).

In addition, an immune response (including CTL) may also be generated by administration of a bacteria which expresses the immunogenic portion(s) discussed above on its cell surface. Representative examples include BCG (Stover, *Nature 351*:456-458, 1991) and salmonella (Newton et al., *Science 244*:70-72, 1989).

Cell mediated and humoral responses may also be induced against hepatitis by parenteral administration of the immunogenic portion(s) discussed above. Briefly, immunogenic portions carrying relevant epitopes can be produced in a number of known ways (Ellis and Gerety, *J. Med. Virol. 31*:54-58, 1990), including chemical synthesis (Bergot et al., *Applied Biosystems Peptide Synthesizer User Bulletin No. 16, 1986,* Applied Biosystems, Foster City California) and DNA expression in recombinant systems, such as the insect-derived baculovirus system (Doerfler, *Current Topics in Immunology 131*:51-68, 1986), mammalian-derived systems (such as CHO cells) (Berman et al., J. *Virol. 63*:3489-3498, 1989), yeast-derived systems (McAleer et al., *Nature 307*:178-180), and prokaryotic systems (Burrel et al., *Nature 279*:43-47, 1979).

The proteins or peptides may then be purified by conventional means and delivered by a number of methods to induce cell-mediated responses, including class I and class II responses. These methods include the use of adjuvants of various types, such as ISCOMS (Morein, *Immunology Letters 25*:281-284, 1990; Takahashi et al., *Nature 344*:873-875m, 1990), liposomes (Gergoriadis et al., *Vaccine 5*:145-151, 1987), lipid conjugation (Deres et al., *Nature 342*:561-564, 1989), coating of the peptide on autologous cells (Staerz et al., *Nature 329*:449-451, 1987), pinosomes (Moore et al., *Cell 54*:777-785, 1988), alum, complete or incomplete Freund's adjuvants (Hart et al., *Proc*. *Natl. Acad. Sci. USA 88*:9448-9452, 1991), or various other useful adjuvants (*e.g.*, Allison and Byars, *Vaccines 87*:56-59, Cold Spring Harbor Laboratory, 1987) that allow effective parenteral administration (Litvin et al., *Advances in AIDS Vaccine Development,* Fifth Annual Meeting of the National Vaccine Development Groups for AIDS, August 30, 1992).

Alternatively, the proteins or peptides corresponding to the immunogenic portion(s) discussed above can be encapsulated for oral administration to elicit an immune response in enteric capsules (Channock et al., *J*. *Amer*. *Med*. *Assoc*. *195*:445-452, 1966) or other suitable carriers, such as poly (DL-lactide-co-glycolate) spheres (Eldridge et al. in Proceedings of the International Conference on Advances in AIDS Vaccine Development, DAIDS, NIAID, U.S. Dept of Health & Human Services, 1991) for gastrointestinal release.

As noted above, immunogenic proteins of the present invention may also be manipulated by a variety of methods known in the art, in order to render them more immunogenic. Representative examples of such methods include: adding amino acid sequences that correspond to T helper epitopes; promoting cellular uptake by adding hydrophobic residues; by forming particulate structures; or any combination of these (*see generally,* Hart, op. cit., Milich et al., *Proc. Natl. Acad. Sci. USA 85*:1610-1614, 1988; Willis, *Nature 340*:323-324, 1989; Griffiths et al., *J. Virol. 65*:450-456, 1991).

Within preferred embodiments of the present invention, pharmaceutical compositions are provided comprising one of the above described recombinant retroviruses in combination with a pharmaceutically acceptable carrier or diluent. The composition may be prepared either as a liquid solution, or as a solid form (*e.g.*, lyophilized) which is suspended in a solution prior to administration. In addition, the composition may be prepared with suitable carriers or diluents for either injection, oral, or rectal administration. Generally, the recombinant virus is utilized at a concentration ranging from 0.25% to 25%, and preferably about 5% to 20% before formulation. Subsequently, after preparation of the composition, the recombinant virus will constitute about 1 ug of material per dose, with about 10 times this amount material (10 µg) as copurified contaminants. Preferably, the composition is prepared in 0.1-1.0 ml of aqueous solution formulated as described below.

Pharmaceutically acceptable carriers or diluents are nontoxic to recipients at the dosages and concentrations employed. Representative examples of carriers or diluents for injectable solutions include water, isotonic saline solutions which are preferably buffered at a physiological pH (such as phosphate-buffered saline or Tris-buffered saline), mannitol, dextrose, glycerol, and ethanol, as well as polypeptides or proteins such as human serum albumin. A particularly preferred composition comprises a vector or recombinant virus in 10 mg/ml mannitol, 1 mg/ml HSA, 20mM Tris, pH 7.2 and 150 mM NaCl. In this case, since the recombinant vector represents approximately 1 µg of material, it may be less than 1% of high molecular weight material, and less than 1/100,000 of the total material (including water). This composition is stable at -70°C for at least six months. The composition may be injected intravenously (i.v.) or subcutaneously (s.c.), although it is generally preferable to inject it intramuscularly (i.m.). The individual doses normally used are 10⁷ to 10⁹ c.f.u. (colony forming units of neomycin resistance titered on HT1080 cells). These are administered at one to four week intervals for three or four doses initially. Subsequent booster shots may be given as one or two doses after 6-12 months, and thereafter annually.

Oral formulations may also be employed with carriers or diluents such as cellulose, lactose, mannitol, poly (DL-lactide-co-glycolate) spheres, and/or carbohydrates such as starch. The composition may take the form of, for example, a tablet, gel capsule, pill, solution, or suspension, and additionally may be formulated for sustained release. For rectal administration, preparation of a suppository may be accomplished with traditional carriers such as polyalkalene glucose, or a triglyceride.

As noted above, the vector construct may direct expression of an immunomodulatory cofactor in addition to at least one immunogenic portion of a hepatitis antigen. If the vector construct, however, does not express an immunomodulatory cofactor which is a cytokine, this cytokine may be included in the above-described compositions, or may be administered separately (concurrently or subsequently) with the above-described compositions. Briefly, within such an embodiment, the immunomodulatory cofactor is preferably administered according to standard protocols and dosages as prescribed in *The Physician's Desk Reference.* For example, alpha interferon may be administered at a dosage of 1-5 million units/day for 2-4 months, and IL-2 at a dosage of 10,000-100,000 units/kg of body weight, 1-3 times/day, for 2-12 weeks. Gamma interferon may be administered at dosages of 150,000-1,500,000 U/m 2-3 times/week for 2-12 weeks.

The following examples are offered by way of illustration and not by way of limitation.

### EXAMPLES

### EXAMPLE 1

### ISOLATION OF HBV E/CORE SEQUENCE

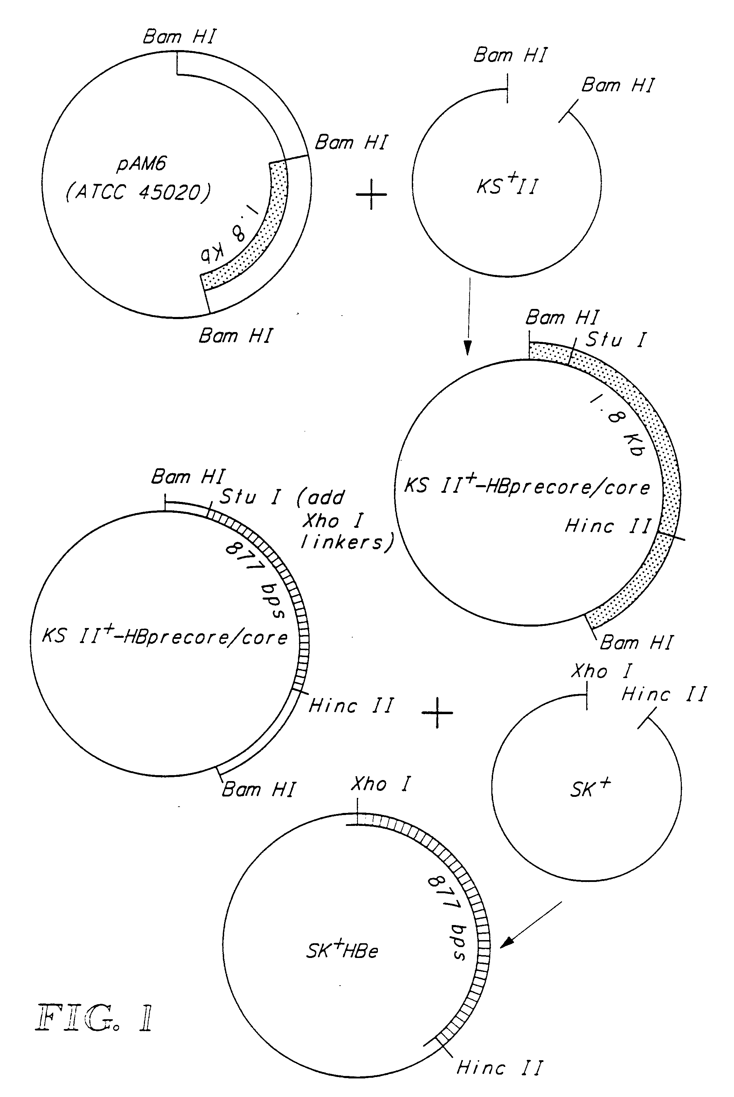

A 1.8 Kb BamH I fragment containing the entire precore/core coding region of hepatitis B is obtained from plasmid pAM6 (ATCC No 45020) and ligated into the BamH I site of KS II⁺ (Stratagene, La Jolla, California). This plasmid is designated KS II⁺ HBpc/c, Figure 1. Xho I linkers are added to the Stu I site of precore/core in KS II⁺ HBpc/c and the resulting 877 base pair Xho I-Hinc II precore/core fragment is cloned into the Xho I/Hinc II site of SK II⁺. This plasmid is designated SK⁺HBe, Figure 1.

### EXAMPLE 2

### PREPARATION OF SEQUENCES UTILIZING PCR

### A. Site-Directed Mutagenesis of HBV e/core Sequences Utilizing PCR

The precore/core gene in plasmid KS II + HB pc/c is sequenced to determine if the precore/core coding region is correct. This sequence was found to have a single base-pair deletion which causes a frame shift at codon 79 that results in two consecutive in-frame TAG stop codons at codons 84 and 85, Figure 2. This deletion is corrected by PCR overlap extension (Ho et al., *Gene 77*:51-59, 1989) of the precore/core coding region in plasmid SK⁺ HBe. Four oligonucleotide primers are used for the 3 PCR reactions performed to correct the deletion.

The first reaction utilizes two primers. The sense primer sequence corresponds to the nucleotide sequence 5 to 27 of the *adw* strain and contains two Xho I restriction sites at the 5' end. The nucleotide sequence numbering is obtained from Genbank (Intelligenics, Inc., Mountain View, California).

The second primer sequence corresponds to the anti-sense nucleotide sequence 2158 to 2130 of the *adw* strain of hepatitis B virus and includes codons 79, 84 and 85.

The second reaction also utilizes two primers. The sense primer which corresponds to nucleotide sequence 2130 to 2158 of the *adw* strain and includes codons 79, 84 and 85.

The second primer corresponds to the anti-sense nucleotide sequence from SK⁺ plasmid polylinker downstream from codons 84 and 85.

The third reaction also utilizes two primers. The sense primer which corresponds to nucleotide sequence 5 to 27 of the *adw* strain and contains two Xho I restriction sites at the 5' end.

The second primer sequence corresponds to the anti-sense nucleotide sequence from the SK⁺ plasmid polylinker downstream from codon 84 and 85.

The first PCR reaction corrects the deletion in the antisense strand and the second reaction corrects the deletion in the sense strands. PCR reactions one and two correct the mutation from CC to CCA which occurs in codon 79 and a base pair substitution from TCA to TCT in codon 81 (*see* Figure 2). Primer 1 contains two consecutive Xho I sites 10 bp upstream of the ATG codon of HBV e coding region and primer 4 contains a Cla I site 135 bp downstream of the stop codon of HBV precore/core coding region. The products of the first and second PCR reactions are extended in a third PCR reaction to generate one complete HBV precore/core coding region with the correct sequence (Figure 3).

The PCR reactions are performed using the following cycling conditions: The sample is initially heated to 94°C for 2 minutes. This step, called the melting step, separates the double-stranded DNA into single strands for synthesis. The sample is then heated at 56°C for 30 seconds. This step, called the annealing step, permits the primers to anneal to the single stranded DNA produced in the first step. The sample is then heated at 72°C for 30 seconds. This step, called the extension step, synthesizes the complementary strand of the single stranded DNA produced in the first step. A second melting step is performed at 94°C for 30 seconds, followed by an annealing step at 56°C for 30 seconds which is followed by an extension step at 72°C for 30 seconds. This procedure is then repeated for 35 cycles resulting in the amplification of the desired DNA product.

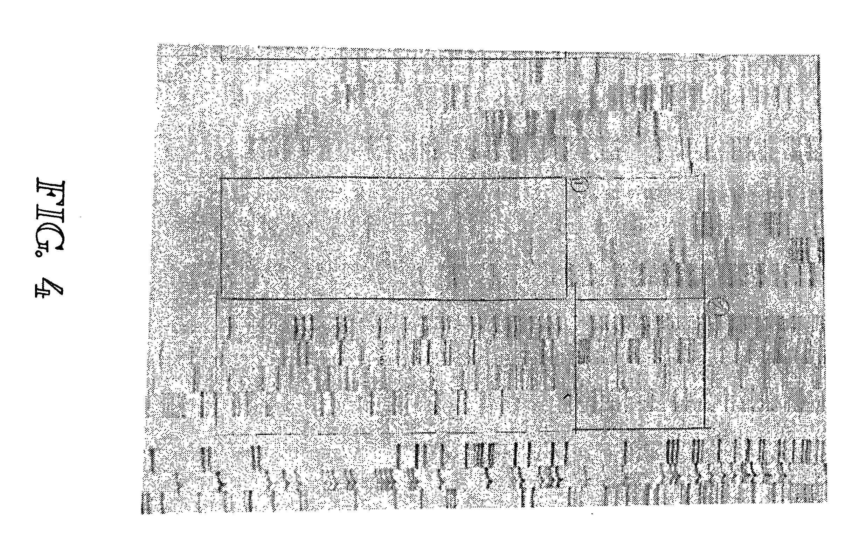

The PCR reaction product is purified by gel electrophoresis and transferred onto NA 45 paper (Schleicher and Schuell, Keene, New Hampshire). The desired 787 bp DNA fragment is eluted from the NA 45 paper by incubating for 30 minutes at 65°C in 400 µl high salt buffer (1.5 M NaCl, 20mM Tris, pH 8.0, and 0.1mM EDTA). Following elution, 500 µl of phenol:chloroform:isoamyl alcohol (25:24:1) is added to the solution. The mixture is vortexed and then centrifuged 14,000 rpm for 5 minutes. The aqueous phase, containing the desired DNA fragment, is transferred to a fresh 1.5 ml microfuge tube and 1.0 ml of 100% EtOH is added. This solution is incubated on dry ice for 5 minutes, and then centrifuged for 20 minutes at 10,000 rpm. The supernatant is decanted, and the pellet is rinsed with 500 µl of 70% EtOH. The pellet is dried by centrifugation at 10,000 rpm under vacuum, and then resuspended in 10 µl deionized H₂O. One microliter of the PCR product is analyzed by 1.5% agarose gel electrophoresis. The 787 base pair Xho I-Cla I precore/core PCR amplified fragment is cloned into the Xho I-Cla I site of SK⁺ plasmid. This plasmid is designated SK⁺HBe-c. *E. coli* (DH5 alpha, Bethesda Research Labs, Gaithersburg, Maryland) is transformed with the SK⁺HBe-c plasmid and propagated to generate plasmid DNA. The plasmid is then isolated and purified, essentially as described by Birnboim et al. (*Nuc. Acid Res. 7*:1513, 1979; *see also Molecular Cloning: A Laboratory Manual*, Sambrook et al. (eds.), Cold Spring Harbor Press, 1989). The SK⁺HB e-c plasmid is analyzed to confirm the sequence of the precore/core gene, Figure 4.

### B. Isolation of HBV core Sequence

The single base pair deletion in plasmid SK⁺ HBe is corrected by PCR overlap extension as described in Example 2A. Four oligonucleotide primers are used for the PCR reactions performed to correct the mutation.

The first reaction utilizes two primers. The sense primer corresponds to the nucleotide sequence for the T-7 promoter of SK⁺HBe plasmid.

The second primer corresponds to the anti-sense sequence 2158 to 2130 of the *adw* strain, and includes codons 79, 84 and 85.

The second reaction utilizes two primers. The anti-sense primer corresponds to the nucleotide sequence for the T-3 promoter present in SK⁺HBe plasmid.

The second primer corresponds to the sense nucleotide sequence 2130 to 2158 of the *adw* strain, and includes codons 79, 84 and 85.

The third reaction utilizes two primers. The anti-sense primer corresponds to the nucleotide sequence for the T-3 promoter present in SK⁺ HBe plasmid.

The second primer corresponds to the sense sequence of the T-7 promoter present in the SK⁺HBe plasmid.

The PCR product from the third reaction yields the correct sequence for HBV precore/core coding region.

To isolate HBV core coding region, a primer is designed to introduce the Xho I restriction site upstream of the ATG start codon of the core coding region, and eliminates the 29 amino acid leader sequence of the HBV precore coding region. In a fourth reaction, the HBV core coding region is produced using the PCR product from the third reaction and the following primers:

The fourth reaction utilizes two primers. The sense primer corresponds to the nucleotide sequence 1885 to 1905 of the *adw* strain and contains two Xho I sites at the 5' end.

The second primer corresponds to the anti-sense nucleotide sequence for the T-3 promoter present in the SK⁺ HBe plasmid. The approximately 600 bp PCR product from the fourth PCR reaction contains the HBV core coding region and novel Xho I restriction sites at the 5' end and Cla I restriction sites at the 3' end that was present in the multicloning site of SK⁺ HBe plasmid.

Following the fourth PCR reaction, the solution is transferred into a fresh 1.5 ml microfuge tube. Fifty microliters of 3 M sodium acetate is added to this solution followed by 500 µl of chloroform:isoamyl alcohol (24:1). The mixture is vortexed and then centrifuged at 14,000 rpm for 5 minutes. The aqueous phase is transferred to a fresh microfuge tube and 1.0 ml 100% EtOH is added. This solution is incubated at -20°C for 4.5 hours, and then centrifuged at 10,000 rpm for 20 minutes. The supernatant is decanted, and the pellet rinsed with 500 µl of 70% EtOH. The pellet is dried by centrifugation at 10,000 rpm under vacuum and then resuspended in 10 µl deionized H₂O. One microliter of the PCR product is analyzed by electrophoresis in a 1.5% agarose gel.

### C. Amplification of Immunomodulatory Cofactor IL-2

Jurkat cells are resuspended at 1 x 10⁶ cells/ml to a total volume of 158 ml in 175 flasks. Phytohemaglutin (PHA) is added to 1% of total volume (1.58 ml total), and incubated overnight at 37°C, 5% CO₂. On the following day, cells are harvested in three 50 ml centrifuge tubes. The three pellets are combined in 50 ml PBS, centrifuged at 3,000 rpm for 5 minutes and supernatant decanted. This procedure is repeated. Poly A⁺ mRNA is isolated using the Micro-Fast Track mRNA Isolation Kit, version 1.2 (Invitrogen, San Diego, California). The isolated intact mRNA is used as the template to generate full-length first strand cDNA by the cDNA CYCLE kit with the following primer.

This oligonucleotide corresponds to the anti-sense nucleotide sequence of the IL-2 mRNA, 25 base pairs downstream of the stop codon.

The product from the reverse transcription reaction is amplified in two separate reactions. The first PCR amplification is performed with the sense primer that corresponds to three bp upstream of the ATG start codon. This primer contains a Hind III site at its 5' end and contains the 5' region of the IL-2 open reading frame including the ATG start codon.

The anti-sense primer is complementary to the 3' region of IL-2 open reading frame and starts three bp downstream of the TGA stop codon. This primer contains an Xho I site at the 5' end of the primer.

The 467 bp PCR product from the first PCR reaction is ligated into the pCR II plasmid, verified by DNA sequencing and designated pCR II H-Xh IL-2

The product from the reverse transcription reaction is amplified in a second PCR reaction. The second PCR amplification is performed with the sense primer that corresponds to three bp upstream of the ATG start codon. This primer contains an Xho I site at its 5' end and contains the 5' region of the IL-2 open reading frame including the ATG start codon.

The anti-sense primer is complementary to the 3' region of IL-2 open reading frame and starts three bp downstream of the TGA stop codon. This primer contains an Apa I site at the 5' end of the primer.

The 467 bp PCR product from the second PCR reaction is ligated into the pCR II plasmid, verified by DNA sequencing and transformed into frozen competent *E. coli* cells. This vector construct is designated pCR II Xh-A IL-2.

### D. Amplification of Immunomodulatory Cofactor B7/BB1 Utilizing PCR

Raji cells are suspended at 1 X 10⁶ cells/ml to a total volume of 158 ml in five T75 flasks and incubated overnight at 37°C, 5% CO₂. On the following day, cells are harvested in three 50 ml centrifuge tubes. Cell pellets are combined in 50 ml PBS, centrifuged at 2,000 rpm for 10 minutes and supernatant decanted. This procedure is repeated. Poly A⁺ mRNA is isolated as described in Example 2E. The isolated intact mRNA is used as the template to generate full-length first strand cDNA using the cDNA CYCLE kit, followed by two separate PCR amplification reactions essentially as described in Example 2E. The nucleotide numbering system is obtained from Freeman et al. (*J. Immunol. 143*:2714-2722, 1989).

The first PCR amplification is performed with two primers. The sense primer corresponds to the nucleotide sequence 315 to 353 of B7/BB1. This primer contains the 5' region of the B7/BB1 open reading frame including the ATG start codon and has two Hind III restriction sites at the 5' end.

The second primer corresponds to the anti-sense nucleotide sequence 1187 to 1149 of B7/BB1. This primer is complementary to the 3' region of the B7/BB1 open reading frame ending at the TAA stop codon and contains two Xho I restriction sites at the 5' end.

The 868 bp PCR product from the first PCR reaction is ligated into the pCR II plasmid, verified by DNA sequencing and transformed into frozen competent *E. coli* cells. This vector construct is designated PCR II H-Xh-B7/BB1 and verified by DNA sequencing.

The second PCR amplification is performed with two primers. The sense primer corresponds to the nucleotide sequence 315 to 353 of B7/BB1. This primer contains the 5' region of the B7/BB1 open reading frame including the ATG start codon and has two Xho I sites at its 5' end.

The second primer corresponds to the anti-sense nucleotide sequence 1187 to 1149 of B7/BB1. This primer is complementary to the 3' region of the B7/BB1 open reading frame ending at the TAA stop codon and contains two Apa I restriction sites at the 5' end.

The 868 bp PCR product from the second PCR reaction is ligated into the pCR II plasmid, verified by DNA sequencing and transformed into frozen competent *E. coli* cells. This vector construct is designated pCR II Xh-A-B7/BB1 and verified by DNA sequencing.

### E. Synthesis of Immunomodulatory Cofactor GM-CSF Utilizing PCR

The synthesis of GM-CSF is performed following the protocol of Foguet and Lubbert (*Biotechniques 13*:674-675, 1992). Ten overlapping oligonucleotides, 53 to 106 nucleotides in length, are synthesized. The first oligonucleotide is the sense sequence of human GM-CSF from nucleotide sequence number 29 to 86 containing two Hind III cleavage sites at the 5' end.

The second oligonucleotide is the sense sequence of human GM-CSF from the nucleotide sequence numbers 29 to 86 containing two Xho I sites at the 5' end.

The third oligonucleotide is the anti-sense sequence of human GM-CSF from nucleotide sequence number 145 to 70.

The fourth oligonucleotide is the sense sequence of human GM-CSF from nucleotide number 131 to 191.

The fifth oligonucleotide is the anti-sense sequence of human GM-CSF from nucleotide number 282 to 176.

The sixth oligonucleotide is the sense sequence of human GM-CSF from nucleotide number 256 to 346.

The seventh oligonucleotide sequence is the anti-sense sequence of human GM-CSF from nucleotide number 331 to 389.

The eighth oligonucleotide is the sense sequence of human GM-CSF from nucleotide number 372 to 431.

The ninth oligonucleotide sequence is the anti-sense sequence of human GM-CSF from nucleotide number 520 to 416 containing two Xho I restriction sites at the 5' end.

The tenth oligonucleotide sequence is identical to oligonucleotide number nine except that it contains two Xba I restriction sites at the 5' terminus instead of Xho I restriction sites.

All the oligonucleotides except for oligonucleotide Sequence ID Nos. 29, 36, 37 and 47 are phosphorylated. Ligation is performed by mixing 8 pmol of each oligonucleotide and 7.5 µl 10X Sequenase Buffer (US Biochemical, Cleveland, Ohio) to a final volume of 75 µl with sterile distilled deionized H₂O. The reaction is heated for 5 minutes at 70°C, followed by 5 minutes at 48°C. Two microliters of dNTP mix (2.5mM each dNTP) and 10 U Sequenase are added and incubated for 30 minutes at 37°C. To inactivate the Sequenase, the ligation reaction is heated for 10 minutes at 70°C (*Current Protocols in Molecular Biology*, F.M. Asubel et al., 8.2.8-8.2.13, 1988).

One microliter of the ligation mixture is used in a PCR reaction with Vent polymerase (New England Biolabs, Beverly, Massachusetts) and the two oligonucleotides Sequence ID Nos. 29 and 36 as primers. The PCR product is ligated into the pCR II vector and transformed into frozen competent *E. coli* cells. This construct is designated pCR II H-Xh GM-CSF and verified by DNA sequencing.

One microliter of the ligation mixture was used in a second PCR reaction with Vent polymerase with the two oligonucleotides Sequence ID Nos. 47 and 37 as primers. The PCR product is ligated into the pCR II vector and transformed into frozen competent *E. coli* cells. This construct is designated pCR II Xh-Xb GM-CSF and verified by DNA sequencing.

### F. Isolation of HBV Pre-S2 Open Reading Frame

The Pre-S2 open reading frame (including S) is PCR amplified with two primers and the pAM 6 plasmid (ATCC No. 45020). The sense primer corresponds to the nucleotides 3178 to 31 of the *adw* strain of hepatitis B virus. The 5' end of the sense primer contains two consecutive Xho I restriction sites. The primer is the 5' region of the Pre-S2 open reading frame and includes the ATG start codon.

The second primer corresponds to the anti-sense nucleotide sequence 907 to 859 and contains two Cla I sites at the 5' end. This primer is complementary to the 3' region of the Pre-Sz open reading frame.

The 957 bp PCR product is ligated into the pCR II plasmid, verified by DNA sequencing and designated pCR II HB-Pre-S2.

### G. Isolation of HBV Polymerase Open Reading Frame

The PCR amplification is performed with two primers and the pAM 6 plasmid (ATCC 40202). The sense primer corresponds to the nucleotides 2309 to 2370 of the *adw* strain of hepatitis B virus. The 5' end of the sense primer contains two consecutive Xho I restriction sites. This primer also contains nucleotide changes to conform to the Kozak rules for translation.

The second primer corresponds to the anti-sense nucleotide sequence 1645 to 1594 and contains two Cla I sites at the 5' end. This primer is complementary to the 3' region of the polymerase open reading frame and includes the TGA stop codon.

The 2564 bp PCR product is ligated into the pCR II plasmid, verified by DNA sequencing and designated pCR II HB-pol.

### H. Isolation of HBV ORF 5 Open Reading Frame

The PCR amplification is performed with two primers and the pAM 6 plasmid (ATCC 45020). The sense primer corresponds to the nucleotides 1432 to 1482 of the *adw* strain of hepatitis B virus. The 5' end of the sense primer contains two consecutive Xho I restriction sites. The primer also contains nucleotide changes to conform to the Kozak rules for translation.

The second primer corresponds to the anti-sense nucleotide sequence 1697 to 1648 and contains two Cla I sites at the 5' end. This primer is complementary to the 3' region of the ORF 5 open rading frame and includes the TAA stop codon.

The 293 bp PCR product is ligated into the pCR II plasmid, verified by DNA sequencing and designated pCR II HB-ORF 5.

### I. Isolation of HBV ORF 6 Open Reading Frame

The PCR amplification is performed with two primers and the pAM 6 plasmid (ATCC 45020). The sense primer corresponds to the nucleotides 1844 to 1788 of the *adw* strain of hepatitis B virus. The 5' end of the sense primer contains two consecutive Xho I restriction sites. The primer also contains nucleotide changes to conform to the Kozak rules for translation.

The second primer corresponds to the anti-sense nucleotide sequence 1188 to 1240 and contains two Cla I sites at the 5' end. This primer is complementary to the 3' ergion of the ORF 6 open reading frame and includes the TAA

The 687 bp PCR product is ligated into the pCR II plasmid, verified by DNA sequencing and designated pCR II HB-ORF 6.

### EXAMPLE 3

### A. Preparation of Retroviral Backbone KT-3

The Moloney murine leukemia virus (MoMLV) 5' long terminal repeat (LTR) EcoR I-EcoR I fragment, including gag sequences, from the N2 vector (Armentano et al., *J. Vir. 61*:1647-1650, 1987; Eglitas et al., *Science 230*:1395-1398, 1985) is ligated into the plasmid SK⁺ (Stratagene, La Jolla, California). The resulting construct is designated N2R5. The N2R5 construct is mutated by site-directed *in vitro* mutagenesis to change the ATG start codon to ATT preventing gag expression. This mutagenized fragment is 200 base pairs (bp) in length and flanked by Pst I restriction sites. The Pst I-Pst I mutated fragment is purified from the SK⁺ plasmid and inserted into the Pst I site of N2 MoMLV 5' LTR in plasmid pUC31 to replace the non-mutated 200 bp fragment. The plasmid pUC31 is derived from pUC19 (Stratagene, La Jolla, California) in which additional restriction sites Xho I, Bgl II, BssH II and Nco I are inserted between the EcoR I and Sac I sites of the polylinker. This construct is designated pUC31/N2R5gM.

A 1.0 Kilobase (Kb) MoMLV 3' LTR EcoR I-EcoR I fragment from N2 is cloned into plasmid SK⁺ resulting in a construct designated N2R3⁻. A 1.0 Kb Cla I-Hind III fragment is purified from this construct. The Cla I-Cla I dominant selectable marker gene fragment from pAFVXM retroviral vector (Kriegler et al., *Cell 38*:483, 1984; St. Louis et al., *PNAS 85*:3150-3154,1988), comprising a SV40 early promoter driving expression of the neomycin phosphotransferase gene, is cloned into the SK⁺ plasmid. A 1.3 Kb Cla I-BstB I gene fragment is purified from the SK⁺ plasmid.

The expression vector is constructed by a three part ligation in which the Xho I-Cla I fragment containing the gene of interest and the 1.0 Kb MoMLV 3' LTR Cla I-Hind III fragment are inserted into the Xho I-Hind III site of pUC31/N2R5gM plasmid. The 1.3 Kb Cla I-BstB I neo gene fragment from the pAFVXM retroviral vector is then inserted into the Cla I site of this plasmid in the sense orientation.

### B. Preparation of Retroviral Backbone KT-1

The KT-1 retroviral backbone vector is constructed essentially as described for KT-3 in Example 3A, with the exception that the dominant selectable marker gene, neo, is not inserted into the expression vector.

### EXAMPLE 4

### CONSTRUCITON OF RETROVIRAL VECTORS

### A. Construction of Hepatitis B Virus e-c Retroviral Vector

The 787 bp Xho I-Cla I fragment from SK⁺ HBe-c, Example 2A, is then ligated into the Xho I and Cla I sites of the KT-3 retroviral vector backbone. This construct is designated KT-HBe-c.

### B. Construction of Hepatitis B Virus core Retroviral Vector

The PCR product from Example 2B, approximately 600 bp in length, is digested with Xho I and Cla I restriction endonucleases, electrophoresed through an 1.5% agarose gel and the DNA is purified from the gel slice by Geneclean II (Bio 101, Vista, California). This Xho I-Cla I HBV core PCR product is inserted into the Xho I and Cla I sites of the KT-3 retroviral vector backbone. The construct is designated KT-HBc.

The HBV core fragment (Xho I-Cla I) from KT-HBc is inserted into the respective sites of pBluescript KS⁺ II (Stratagene, La Jolla, California). This construct is designated KS⁺ II HBc, and is verified by DNA sequencing.

### C. Construction of Hepatitis B Virus Pre-S2 Retroviral Vector

The Xho I-Cla I fragment from PCR II Pre-S2 is excised and inserted into the respective sites of the KT3 backbone. This construct is designated KT-HB-Pre-S2.

### D. Construction of Hepatitis B Virus Polymerase Retroviral Vector

The Xho I-Cla I fragment from pCR II HB-pol is excised and inserted into the respective sites of the KT3 backbone. This construct is designated KT-HB-pol.

### E. Construction of Hepatitis B Virus ORF 5 Retroviral Vector

The Xho I-Cla I fragment from pCR II HB-ORF-5 is excised and inserted into the respective sites of the KT3 backbone. This construct is designated KT-HB-ORF 5.

### F. Construction of Hepatitis B Virus ORF 6 Retroviral Vector

The Xho I-Cla I fragment from pCR II HB-ORF-6 is excised and inserted into the respective sites of the KT3 backbone. This construct is designated KT-HB-ORF 6.

### EXAMPLE 5

### CONSTRUCTION OF MULTIVALENT RETROVIRAL VECTOR

### A. Construction of Hepatitis B e/GM-CSF Retroviral Vector

### i. Multivalent retroviral vector with IRBS

pGEM 5Z⁺BIP 5' (Peter Sarnow, University of Colorado, Health Sciences Center, Denver, human immunoglobulin heavy chain binding protein) is digested with Sac I and Sph I. The 250 bp BIP fragment is isolate by 1.5% agarose gel electrophoresis and subcloned into the respective sites of pSP72. The vector construct is designated pSP72 BIP.

The Hind III-Xho I GM-CSF fragment is excised from pCR II H-Xh GM-CSF and subcloned into the Hind III-Xho I sites of pSP72 BIP. This construct is designated pSP72 BIP-GM-CSF.

The construct pSP72 BIP GM-CSF is cleaved at the Xho I site and blunted by Klenow fragment, followed by cleavage with Cla I. The KT-1 backbone is cleaved by Cla I and blunted with Klenow fragment followed by cleavage with Xho I restriction endonuclease. In a three-part ligation, the Xho I-Cla I fragment from SK⁺ HBe-c, Example 2A, and the Cla I-blunted Xho I BIP-GM-CSF fragment is ligated into the Xho I-blunted Cla I sites of the KT-1 retroviral backbone. This construct is designated KT-HBe-c/BIP-GM-CSF.

### ii. Multivalent retroviral vector with CMV promoter

The 4.7 Kb CMV Env^{R} Pst-RI fragment is isolated from pAF/CMV/Env^{R} (U.S. Patent Application No. 07/395,932), and inserted into the Pst I and Eco RI sites of pUC 18. This construct is designated pUC 18 CMV Env^{R}.

HIV-1 III_{B} CAR is subcloned as a Sau 3A fragment from pAF/CMV/Env^{R} into the BamH I site of pBluescript II KS⁺ (Stratagene, La Jolla, California) to generate pBluescript II KS⁺/CAR. The CAR fragment is excised from pBluescript II KS⁺/CAR as a Xba I-Cla I fragment. The Xho I-Xba I HIV-1 III_{B} gag/pol fragment is excised from SK⁺ gag/pol SD delta (U.S. Patent Application No. 07/395,932). The plasmid backbone containing the CMV promoter is excised from pUC18 CMV/Env^{R} with Xho I and Cla I. In a three part ligation, the Xho I-Xba I HIV III_{B} gag-pol fragment and the Xba I-Cla I CAR fragment is inserted into the Xho I - Cla I sites of the pUC 18 CMV/Env^{R} backbone to generate pUC 18 CMV gag/pol/CAR.

The Hind III-Xho I fragment containing the CMV IE promoter from pUC 18 CMV-gag/pol/CAR is subcloned into the respective sites of pCDNA II. This construct is designated pCDNA II CMV.

The Xho I-Xba I GM-CSF PCR product is subcloned from the pCR II Xh-Xb GM-CSF and inserted into the respective sites within pCDNA II-CMV; This construct is designated pCDNA II CMV-GM-CSF.

The pCDNA II CMV GM-CSF construct is cleaved at the Xba I site, blunted by Klenow fragment, followed by cleavage with Hind III. The KT-1 backbone is cleaved by Cla I and blunted with Klenow fragment followed by cleavage with Xho I. In a three-part ligation, the Xho I-Hind III fragment from SK⁺HBe-c, Example 2A, and the Hind III-blunted Xba I CMV-GM-CSF fragment is ligated into the Xho I-blunted Cla I sites of the KT-1 retroviral backbone. This vector construct is designated KT-HBe-c/CMV-GM-CSF.

### B. Construction of Hepatitis C core/IL-2 Retroviral Vector

### i. Multivalent retroviral vector with IRBS

The Hind III-Xho I IL-2 sequence is excised from pCR II H-Xh IL-2 and subcloned into the Hind III-Xho I sites of pSP72 BIP. This construct is designated pSP72 BIP IL-2. The Xho I-Hind III hepatitis C virus core sequence, Example 2C, is excised from pCRII Xh-H HCV C core and subcloned into the respective sites of pSP72. This construct is designated pSP72 Xh-H HCV core.

The construct pSP72 BIP-IL2 is cleaved at the Xho I site, blunted by Klenow fragment followed by cleavage with Eco RI. The Xho I-Eco RI HCV core fragment is isolated from pSP72 Xh-H HCV core. The KT-1 backbone is cleaved by Cla I and blunted with Klenow fragment followed by cleavage with Xho I. In a three-part ligation, the Xho I-Eco RI HCV core fragment and the Eco RI-blunted Xho I BIP-IL2 fragment is ligated into the Xho I-blunted Cla I sites of the KT-1 retroviral backbone. This vector construct is designated KT-HCV core/BIP-IL2.

### ii. Multivalent retroviral vector with CMV promoter

The Xho I-Apa I IL-2 fragment is excised from pCR II Xh-A IL-2 and subcloned into the respective sites of pCDNA II-CMV promoter. This construct is designated pCDNA II CMV-IL-2.

The KT-1 backbone is cleaved by Cla I and blunted with Klenow fragment followed by cleavage with Xho I. The construct pCDNA II CMV-IL-2 is cleaved at the Apa I site, blunted by Klenow fragment and followed by cleavage with Hind III restriction endonuclease. In a three-part ligation, the Xho I-Hind III HCV core fragment from pCR II Xh-H HCV core and the Hind III-blunted Apa I CMV IL-2 fragment is ligated into the Xho I-blunted Cla I sites of the KT-1 retroviral backbone. This vector construct is designated KT-HCV core/CMV IL-2.

### C. Construction of Hepatitis B core/B7/BB1 Retroviral Vector

### i. Multivalent retroviral vector with IRBS

The Hind III-Xho I B7/BB1 sequence is excised from pCR II H-Xh B7/BB1 and subcloned into the Hind III-Xho I sites of pSP72 BIP. This construct is designated pSP72 H-Xh BIP-87/BB1.

The construct pSP72 H-Xh BIP-B7/BB1 is cleaved at the Xho I site, blunted by Klenow fragment followed by cleavage with Cla I. The Xho I-Cla I HBV core fragment is isolated from KS II⁺ HBV core, Example 5B. The KT-1 backbone is cleaved by Cla I and blunted with Klenow fragment followed by cleavage with Xho I. In a three-part ligation, the Xho I-Cla I HBV core fragment and the Cla I-blunted Xho I BIP-B7/BB1 fragment is ligated into the Xho I-blunted Cla I sites of the KT-1 retroviral backbone. This vector construct is designated KT-HBV core/BIP-B7/BB1, Example 7.

### ii. Multivalent retroviral vector with CMV promoter

The Xho I-Apa I B7/BB1 sequence is excised from pCR II Xh-A B7/BB1 and subcloned into the respective sites of pCDA II-CMV promoter. This construct is designated pCDNA II CMV-B7/BB1.

The KT-1 backbone is cleaved by Cla I and blunted with Klenow fragment followed by cleavage with Xho I. The construct pCDNA II CMV-B7/BB1 is cleaved at the Apa I site blunted by Klenow fragment and followed by cleavage with HIND III restriction endonuclease. In a three-part ligation, the Xho I-Hind III HBV core fragment from KSII⁺ HBV core and the Hind III-blunted Apa I CMV B7/BB1 fragment is ligated into the Xho I-blunted Cla I sites of the KT-1 retroviral backbone. This vector construct is designated KT-HBV core/CMV B7/BB1

### D. Construction of Hepatitis B e/Hepatitis C core Retroviral Vector

### i. Multivalent retroviral vector with IRBS

The Hind III-Xho I HCV core PCR product is subcloned from the pCR II H-Xh HCV core, Example 2C, and inserted into the respective sites within pSP72-BIP. This construct is designated pSP72 BIP-HCV core.

The construct pSP72 BIP-HCV core is cleaved at the Xho I site, blunted by Klenow fragment, followed by cleavage with Cla I. The KT-1 backbone is cleaved by Cla I and blunted with Klenow fragment followed by cleavage with Xho I. In a three part ligation, the Xho I-Cla I HBV e fragment from SK⁺ HBe-c, Example 2A, and the Cla I-blunted Xho I BIP HCV core fragment is ligated into the Xho I-blunted Cla I sites of the KT-1 retroviral backbone. This vector construct is designated KT-HBV e/BIP HCV core.

### ii. Multivalent retroviral vector with CMV promoter

The Xho I-Xba I HCV core fragment from pSP72 Xh-H HCV core (Example 6B i) is inserted into the respective sites of pCDNA II CMV plasmid. This construct is designated pCDNA II CMV HCV core.

The construct pCDNA II CMV HCV core is cleaved at the Xba I site, blunted by Klenow fragment, followed by cleavage with Hind III. The KT-1 backbone is cleaved by Cla I and blunted with Klenow fragment followed by cleavage with Xho I. In a three part ligation, the Xho I-Hind III HBV e sequence from SK⁺HBe-c, Example 2A, the Hind III-blunted Xba I CMV HCV core fragment is ligated into the Xho I-blunted Cla I sites of the KT-1 retroviral backbone. This vector construct is designated KT-HBVe/CMV HCV core.

### EXAMPLE 6

### TRANSIENT TRANSFECTION AND TRANSDUCTION OF PACKAGING CELL LINES HX AND DA

### A. Plasmid DNA Transfection

DX cells (W092/05266) are seeded at 5 x 10⁵ cells on a 10 cm tissue culture dish on day 1 with Dulbecco's Modified Eagle Medium (OMEM) and 10% Fetal Bovine Serum (FBS). On day 2, the media is replaced with 5.0 ml fresh media 4 hours prior to transfection. A standard calcium phosphate-DNA coprecipitation is performed by mixing 40.0 µl 2.5 M CaCl₂, 10 µg plasmid DNA and deionized H₂O to a total volume of 400 µl. Four hundred microliters of the DNA-CaCl₂ solution is added dropwise with constant agitation to 400 µl precipitation buffer (50 mM HEPES-NaOH, pH 7.1; 0.25 M NaCl and 1.5 mM Na₂HPO₄-NaH₂PO₄). This mixture is incubated at room temperature for 10 minutes. The resultant fine precipitate is added to a culture dish of cells. The cells are incubated with the DNA precipitate overnight at 37°C. On day 3 the media is aspirated and fresh media is added. The supernatant containing virus is removed on day 4, passed through a 0.45 µ filter and used to infect the DA packaging cell line, murine fibroblasts or stored at -80°C.

### B. Packaging Cell Line Transduction

DA (W092/05266) cells are seeded at 5x10⁵ cells/10 cm tissue culture dish in 10 ml DMEM and 10% FBS 4 µg/ml polybrene (Sigma, St. Louis, Missouri) on day 1. On day 2, 3.0 ml, 1.0 ml and 0.2 ml of the freshly collected virus containing DX media is added to the cells. The cells are incubated with the virus overnight at 37°C. On day 3 the media is removed and 1 ml DMEM, 10% FBS with 800 µg/ml G418 is added to the plate. Only cells that have been transfected with the vector and contain the neo selectable marker will survive. A G418 resistant pool is generated over a period of a week. The pool is tested for expression as described (Example 10). The pool of cells is dilution cloned by removing the cells from the plate and counting the cell suspension, diluting the cells suspension down to 10 cells/ml and adding 0.1 ml to each well (1cell/well) of a 96 well plate. Cells are incubated for 14 days at 37°C, 10% CO₂. Twenty-four clones are selected and expanded up to 24 well plates, 6 well plates then 10 cm plates at which time the clones are assayed for expression and the supernatants are collected and assayed for viral titer.

The titer of the individual clones is determined by infection of HT1080 cells, human fibroblast cell line ATCC CCL 121. On day 1, 5x10⁵ HT1080 cells are plated on each well of a 6 well microtiter plate in 3.0 ml DMEM, 10% FBS and 4 µg/ml polybrene. The supernatant from each clone is serially diluted 10 fold and used to infect the HT1080 cells in 1.0 ml aliquots. The cells are incubated with the vector overnight 37°C, 10% CO₂ and the media is replaced with fresh DMEM, 10% FBS media on day 2. On day 3, selection of transduced cells is performed by replacing the media with fresh DMEM, 10% FBS media containing 800 µg/ml G418. Cells are incubated at 37°C, 10% CO₂ for 14 days at which time G418 resistant colonies are scored at each dilution to determine the viral titer of each clone as colony forming units/ml (cfu/ml).

Using these procedures it can be shown that the titers of the HBVcore and HBVe producer cell lines are:

| | |
|---|---|
| DAcore-1 | 8x10⁵ cfu/ml |
| DAcore-10 | 1x10⁶ cfu/ml |
| DAHBe 4-7 | 3x10⁶ cfu/ml |

The packaging cell line HX, WO 92/05266, is transduced with vector generated from the DA vector producing cell line in the same manner as described for transduction of the DA cells from DX supernatant.

For transduction of the DA (WO 92/05266) cells with a muitivalent vector, lacking a neo selectable marker, the infection procedure as noted above is used. However, instead of adding G418 to the cells on day 3, the cells are cloned by limiting dilution as explained above. Fifty clones are expanded for expression as explained above, and titer assayed as described in Example 8.

### EXAMPLE 7

### DETECTION OF REPLICATION COMPETENT RETROVIRUSES

The extended S⁺L⁻ assay determines if replication competent, infectious virus is present in the supernatant of the cell line of interest. The assay is based on the empirical observation that infectious retroviruses generate foci on the indicator cell line MiCl₁ (ATCC CCL 64.1). The MiCl₁ cell line is derived from the Mv1Lu mink cell line (ATCC CCL 64) by transduction with Murine Sarcoma Virus (MSV). It is a non-producer, non-transformed, revertant clone containing a murine sarcoma provirus that forms sarcoma (S⁺) indicating the presence of the MSV genome but does not cause leukemia (L⁻) indicating the absence of replication competent virus. Infection of MiCl₁ cells with replication competent retrovirus "activates" the MSV genome to trigger "transformation" which results in foci formation.

Supernatant is removed from the cell line to be tested for presence of replication competent retrovirus and passed through a 0.45 µ filter to remove any cells. On day 1 Mv1Lu cells are seeded at 1x10⁵ cells per well (one well per sample to be tested) of a 6 well plate in 2 ml DMEM, 10% FBS and 8 µg/ml polybrene. Mv1Lu cells are plated in the same manner for positive and negative controls on separate 6 well plates. The cells are incubated overnight at 37°C, 10% CO₂. On day 2, 1.0 ml of test supernatant is added to the Mv1Lu cells. The negative control plates are incubated with 1.0 ml of media. The positive control consists of three dilutions (200 focus forming units, (ffu), 20 ffu and 2 ffu each in 1.0 ml media) of MA virus (Miller et al., *Molec. and Cell. Biol. 5*:431-437, 1985) which is added to the cells in the positive control wells. The cells are incubated overnight. On day 3 the media is aspirated and 3.0 ml of fresh DMEM and 10% FBS is added to the cells. The cells are allowed to grow to confluency and are split 1:10 on day 6 and day 10, amplifying any replication competent retrovirus. On day 13 the media on the Mv1Lu cells is aspirated and 2.0 ml DMEM and 10% FBS is added to the cells. In addition the MiCl₁ cells are seeded at 1x10⁵ cells per well in 2.0 ml DMEM, 10% FBS and 8 µg/ml polybrene. On day 14 the supernatant from the Mv1Lu cells is transferred to the corresponding well of the MiCl₁ cells and incubated overnight at 37°C, 10% CO₂. On day 15, the media is aspirated and 3.0 ml of fresh DMEM and 10% FBS is added to the cells. On day 21 the cells are examined under the microscope at 10X power for focus formation (appearing as clustered, refractile cells that overgrow the monolayer and remain attached) on the monolayer of cells. The test article is determined to be contaminated with replication competent retrovirus if foci appear on the MiCl₁ cells.

Using these procedures, it can be shown that the HBV core producer cell lines DA core-1, DA core-10, and HBVe producer cell line DA HBe 4-7, are not contaminated with replication competent retroviruses.

### EXAMPLE 8

### TITERING OF MULTIVALENT VECTORS

Since the multivalent vectors do not contain a selectable marker, such as the neomycin gene, another way of titering the vector is described. More specifically, 1.0 ml of vector supernatant is diluted five fold to a final dilution of 10⁻⁹ ml. One milliliter of each dilution is then used to transduce 5 x 10⁵ HT1080 cells (ATCC No. CCL 121) essentially as noted in Example 7B. However, instead of adding G418, DNA is extracted from each dish 7 days later as described by Willis (*J. Biol. Chem. 259*:7842-7849, 1984). The HBV e/core is amplified by PCR using the following PCR primers obtained from Genset (Paris, France).

The PCR amplification for HBV e/core is performed with the sense primer that corresponds to the nucleotide sequence 1865 to 1889 of the *adw* clone.

This primer corresponds to the anti-sense nucleotide sequence 2430 to 2409 of the *adw* clone.

The probe sequence used to confirm the presence of the desired PCR product and corresponds to the nucleotide sequence 1926 to 1907 of the *adw* strain of hepatitis B virus.

The PCR products are analyzed by Southern blot analysis with the appropriate ³²P-labeled probes (Sambrook et al., *Molecular Cloning, a Laboratory Manual,* 2nd ed., Cold Spring Harbor Laboratory, Cold Spring Harbor, NY, 1989). Signal is expected in all of the lower dilutions and gradually decrease at higher dilutions. The last dilution where a signal is visible yields the infectious U/ml of the vector.

### EXAMPLE 9

### A. Transduction of Murine Cells with Vector Construct

The murine fibroblast cell lines BC10ME (ATCC No. TIB85) B16 and L-M(TK⁻) (ATCC No. CCL 1.3) are grown in DMEM containing 4500 mg/L glucose, 584 mg/L L-glutamine (Irvine Scientific, Santa Ana, California) and 10% fetal bovine serum (FBS) (Gemini, Calabasas, California).

The BC10ME, B16, and L-M(TK⁻) fibroblast cell lines are plated at 1x10⁵ cells each in a 10 cm dish in DMEM, 10% FBS complete and 4 µg/ml polybrene. Each is transduced with 1.0 ml of the retroviral vector having a vector titer of approximately 10⁵ cfu/ml. Clones are selected in DMEM, 10% FBS and 800 µg/ml G418 as described in Example 6B.

The EL4 (ATCC No. TIB 39) cells and EL4/A2/K^{b} cells (Sherman, L. Scripps Institute, San Diego, California) are transduced by co-culture with the DA producer cells. Specifically, 1.0 x 10⁶ EL4 cells or 1 x 10⁶ EL4/A2/Kb are added to 1x10⁶ irradiated (10,000 rads) DA (vector titer of approximately 10⁵-10⁶) producer cells in RPMI 1640 (Irvine Scientific, Santa Ana, California), 10% FBS, and 4 µg/ml polybrene (Sigma, St. Louis, Missouri) on day 1. On day 2, 1.0 x 10⁶ irradiated (10,000 rad) DA producer cells are added to the co-culture. On day 5 selection of the transduced EL4 or EL4/A2/KB cells is initiated with 800 µg/ml G418. The pool is dilution cloned as described in Example 6B.

BC10ME, B16, L-M(TK⁻), EL-4 cells transduced by multivalent vectors are not selected in G418; they are cloned by limiting dilution as in Example 7B and assayed for expression as described in Example 10A.

### B. Transduction of Human Cells with Vector Construct

Lymphoblastoid cell lines (LCL) are established for each patient by infecting (transforming) their B-cells with fresh Epstein-Barr virus (EBV) taken from the supernatant of a 3-week-old culture of B95-8, EBV transformed marmoset leukocytes (ATCC CRL 1612). Three weeks after EBV-transformation, the LCL are transduced with retroviral vector expressing HBV core or e antigen. Transduction of LCL is accomplished by co-culturing 1.0 x 10⁶ LCL cells with 1.0 x 10⁶ irradiated (10,000 rads) HX producer cells in a 6 cm plate containing 4.0 ml of medium and 4.0 mg/ml polybrene. The culture medium consists of RPMI 1640, 20% heat inactivated fetal bovine serum (Hyclone, Logan, Utah), 5.0 mM sodium pyruvate and 5.0 mM non-essential amino acids. After overnight co-culture at 37°C and 5% CO₂, the LCL suspension cells are removed and 1 x 10⁶ cells are again co-cultured for another 6-18 hours in a fresh plate containing 1.0 x 10⁶ irradiated (10,000 rads) HX producer cells. Transduced LCL cells are selected by adding 800 mg/ml G418 and cloned to obtain high expression clones. The Jurkat A2/K^{b} cells (L. Sherman, Scripps Institute, San Diego, California) are transduced essentially as described for the transduction of LCL cells. LCLs transduced by multivalent vectors are not selected in G418; they are cloned by limiting dilution as in Example 6B and assayed for expression as in Example 10A.

### EXAMPLE 10

### EXPRESSION OF TRANSDUCED GENES

### A. ELISA

Cell lysates from cells transduced by KT-HBe or KT-HBc are made by washing 1.0 x 10⁷ cultured cells with PBS, resuspending the cells to a total volume of 600 µl on PBS, and sonicating for two 5-second periods at a setting of 30 in a Branson sonicator, Model 350, (Fisher, Pittsburgh, Pennsylvania) or by freeze thawing three times. Lysates are clarified by centrifugation at 10,000 rpm for 5 minutes.

Core antigen and precore antigen in cell lysates and secreted e antigen in culture supernatant are assayed using the Abbott HBe, rDNA EIA kit (Abbott Laboratories Diagnostic Division, Chicago, Illinois). Another sensitive EIA assay for precore antigen in cell lysates and secreted e antigen in culture supernatant is performed using the Incstar ETI-EB kit, (Incstar Corporation, Stillwater, MN). A standard curve is generated from dilutions of recombinant hepatitis B core and e antigen obtained from Biogen (Geneva, Switzerland).

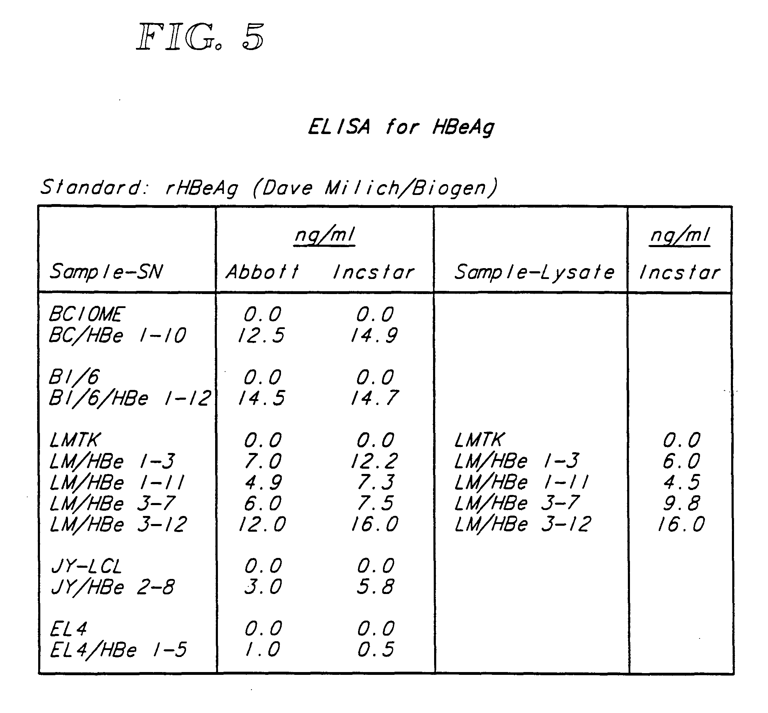

Using these procedures approximately 10 ng/ml e antigen is expressed in transduced cell lines that have been prepared as described in paragraph 1 of this example (see Figure 5).

### B. Expression of Transduced Genes by Western Blot Analysis

Proteins are separated according to their molecular weight (MW) by means of SDS polyacrylamide gel electrophoresis. Proteins are then transferred from the gel to a IPVH Immobilon-P membrane (Millipore Corp., Bedford, Massachusetts.). The Hoefer HSI TTE transfer apparatus (Hoefer Scientific Instruments, California) is used to transfer proteins from the gel to the membrane. The membrane is then probed with polyclonal antibodies from patient serum that reacts specifically with the expressed protein. The bound antibody is detected using ¹²⁵I-labeled protein A, which allows visualization of the transduced protein by autoradiography.

### C. Immunoprecipitation/Western Blot

Characterization of the precore/core and e antigens expressed by transduced cells is performed by immunoprecipitation followed by Western blot analysis. Specifically, 0.5-1.0 ml of cell lysate in PBS or culture supernatant is mixed with polyclonal rabbit anti-hepatitis B core antigen (DAKO Corporation, Carpinteria, California) bound to G-Sepharose (Pharmacia LKB, Uppsala, Sweden) and incubated overnight at 4°C. Samples are washed twice in 20 mM Tris-HCl, pH 8.0, 100 mM NaCl, 10 mM EDTA and boiled in sample loading buffer with 05% 2-beta mercaptoethanol. Proteins are transferred to Immobilon (Millipore Corp., Bedford, Maine) and probed with the DAKO polyclonal rabbit anti-hepatitis core antigen followed by ¹²⁵I-protein A.

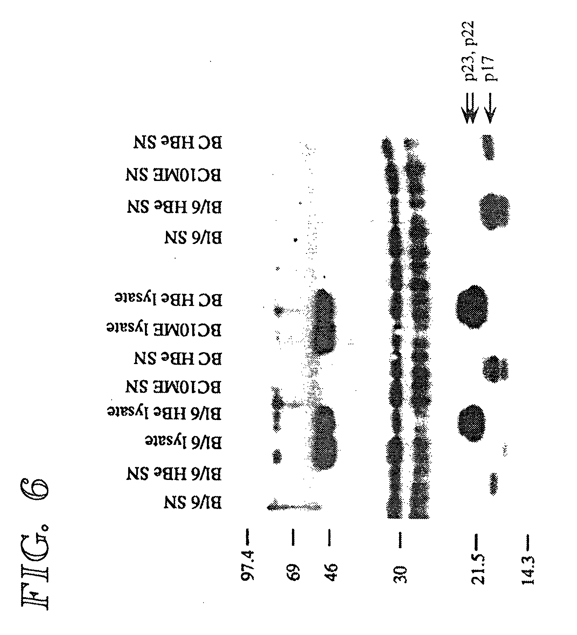

Using these procedures, it can be shown that the 17 Kd HB e protein is secreted by transduced mouse cells into the culture supernatant and the p22, p23 intermediate hepatitis B e products are present mainly the lysates of transduced mouse cells, Figure 6.

### EXAMPLE 11

### A. Cytotoxicity Assays

### i. Inbred Mice

Six- to eight-week-old female Balb/c, C57B1/6 and C3H mice (Harlan Sprague-Dawley, Indianapolis, Indiana) are injected twice intraperitoneally (i.p.) with 1 x 10⁷ irradiated (10,000 rads at room temperature) vector transduced BC10ME, Bl6 and L-M(TK⁻) cells respectively. Animals are sacrificed 7 days later and the splenocytes (3 x 10⁶/ml) cultured *in vitro* with their respective irradiated transduced cells (6 x 10⁴/ml) in T-25 flasks (Corning, Corning, New York). Culture medium consists of RPMI 1640, 5% heat-inactivated fetal bovine serum, 1 mM sodium pyruvate, 50 µg/ml gentamycin and 10⁻⁵M β 2-mercaptoethanol (Sigma, St. Louis, Missouri). Effector cells are harvested 4-7 days later and tested using various effector:target cell ratios in 96 well microtiter plates (Corning, Corning, New York) in a standard chromium release assay. Targets are the transduced and non-transduced BC10ME, Bl6 and L-M(TK⁻) where the non-transduced cell lines are used as negative controls. Specifically, Na₂⁵¹CrO₄-labeled (Amersham, Arlington Heights, Illinois)(100 uCi, 1 hr at 37°C) target cells (1 x 10⁴ cells/well) are mixed with effector cells at various effector to target cell ratios in a final volume of 200 µl. Following incubation, 100 µl of culture medium is removed and analyzed in a Beckman gamma spectrometer (Beckman, Dallas, Texas). Spontaneous release (SR) is determined as CPM from targets plus medium and maximum release (MR) is determined as CPM from targets plus 1M HCl. Percent target cell lysis is calculated as: [(Effector cell + target CPM) - (SR)/(MR) - (SR)] x 100. Spontaneous release values of targets are typically 10%-20% of the MR.

### ii. HLA A2.1 Transgenic Mice

Six- to eight-week-old female HLA A2.1 transgenic mice (V. Engelhard, Charlottesville, Virginia) are injected twice intraperitoneally (i.p.) with 1 x 10⁷ irradiated (10,000 rads at room temperature) vector transduced EL4 A2/Kb cells. Animals are sacrificed 7 days later and the splenocytes (3 x 10⁶/ml) cultured *in vitro* with irradiated (10,000 rads) transduced Jurkat A2/K^{b} cells (6 x 10⁴/ml) in flasks (T-25, Corning, Corning, New York). The remainder of the chromium release assay is performed as described in Example 12A, where the targets are transduced and non-transduced EL4 A2/K^{b} and Jurkat A2/K^{b} cells. Non-transduced cell lines are utilized as negative controls.

### iii. Human CTL assays

Human PBMC are separated by Ficoll (Sigma, St. Louis, Missouri) gradient centrifugation. Specifically, cells are centrifuged at 3,000 rpm at room temperature for 5 minutes. The PBMCs are restimulated *in vitro* with their autologous transduced LCL, Example 10B, at an effector:target ratio of 10:1 for 10 days. Culture medium consists of RPMI 1640 with prescreened lots of 5% heat-inactivated fetal bovine serum 1 mM sodium pyruvate and 50 µn/ml gentamycin. The resulting stimulated CTL effectors are tested for CTL activity using transduced autologous LCL or HLA matched cells as targets in the standard chromium release assay, Example 11A. Since most patients have immunity to EBV, the non-transduced EBV-transformed B-cells (LCL) used as negative controls, will also be recognized as targets by EBV-specific CTL along with the transduced LCL. In order to reduce high background cytotoxicity due to killing of labeled target cells by EBV-specific CTL, it is necessary to add unlabeled non-transduced LCL to labeled target cells at a ratio of 50:1.

### B. Detection of Humoral Immune Response

Humoral immune responses specific for HBV core and e antigens are detected by ELISA. The ELISA protocol utilizes 100 µg/well of recombinant HBV core and recombinant HBV e antigen (Biogen, Geneva, Switzerland) to coat 96-well plates. Sera from mice immunized with cells or direct vector expressing HBV core or HBV e antigen are then serially diluted in the antigen-coated wells and incubated for 1 to 2 hours at room temperature. After incubation, a mixture of rabbit anti-mouse IgG1, IgG2a, IgG2b, and IgG3 with equivalent titers is added to the wells. Horseradish peroxidase ("HRP")-conjugated goat anti-rabbit antiserum is added to each well and the samples are incubated for 1 to 2 hours at room temperature. After incubation, reactivity is visualized by adding the appropriate substrate. Color will develop in wells that contain antibodies specific for HBV core or HBV e antigen.

Using these procedures, it can be shown that antibody to HBV core and e antigens can be induced, Figures 6A and 6B.

### C. T cell proliferation

Antigen induced T-helper activity resulting from two or three injections of direct vector preparations expressing HBV core or e antigen, is measured *in vitro.* Specifically, splenocytes from immunized mice are restimulated *in vitro* of a predetermined ratio with cells expressing HBV core or e antigen or with cells not expressing HBV core or e antigen as a negative control. After five days in RPMI 1640 culture medium containing 5% FBS, 1.0 mM sodium pyruvate and 10⁻⁵ 2-beta mercaptoethanol at 37°C and 5% CO₂, supernatant is tested for IL-2 activity, which is secreted specifically by T-helper cells stimulated by HBV core or e antigen. IL-2 activity is measured using the CTL clone, CTLL-2 (ATCC TIB 214), which is dependent on IL-2 for growth. The CTLL-2 clone will not proliferate in the absence of IL-2. CTLL-2 cells are added to serial dilutions of supernatant test samples in a 96-well plate and are incubated at 37°C and 5%, CO₂ for 3 days. Subsequently, 0.5 µCi ³H-thymidine is added to the CTLL-2. ³H-thymidine is incorporated only if the CTLL-2 cells proliferate. After an overnight incubation, cells are harvested using a PHD cell harvester (Cambridge Technology Inc., Watertown, Massachusetts) and counted in a Beckman beta counter. The amount of IL-2 in a sample is determined from a standard curve generated from a standard recombinant IL-2 obtained from Boehringer Mannheim, Indianapolis, Indiana).

### EXAMPLE 12

### IDENTIFICATION OF IMMUNOGENIC DOMAINS OF HBV PRECORE/CORE

T-cell epitopes may be predicted utilizing computer algorithms such as T-Sites (MedImmune, Maryland). From this analysis, peptides are synthesized and used to identify CTL epitopes. Effector cells from individuals with acute hepatitis B infection that have been stimulated *in vitro* with transduced autologous (Example 10B) LCL are tested on autologous LCLs coated with the peptide. The chromium release assay is performed as described in Example 12A iii, except that peptide is added to non-transduced Na₂⁵¹CrO₄-labeled LCL along with effector cells to a final concentration of 1-100 µg/ml. The reaction is incubated 4-6 hours and a standard chromium release assay performed as described in Example 11A i.

### EXAMPLE 13

### TUMORIGENICITY AND TRANSFORMATION

### A. Tumorigenicity Assay

Tumor formation in nude mice is a particularly important and sensitive method for determining tumorigenicity. Nude mice do not possess mature T-cells, and therefore lack a functional cellular immune system, providing a useful *in vivo* model in which to test the tumorigenic potential of cells. Normal non-tumorigenic cells do not display uncontrolled growth properties if injected into nude mice. However, tumorigenic transformed cells will rapidly proliferate and generate tumors in nude mice. Briefly, the vector construct is administered by injection into nude mice. The mice are visually examined for a period of 4 to 16 weeks after injection in order to determine tumor growth. The mice may also be sacrificed and autopsied in order to determine whether tumors are present (Giovanella et al., *J. Natl. Cancer Inst. 48*:1531-1533, 1972; Furesz et al., "Tumorigenicity testing of cell lines considered for production of biological drugs," *Abnormal Cells,* New Products and Risk, Hopps and Petricciani (eds), Tissue Culture Association, 1985; Levenbook et al., *J. Blot Std. 13*:135-141, 1985). This test is performed by Quality Biotech Inc., (Camden, New Jersey).

### B. Transformation Assay

Tumorigenicity has shown to be closely correlated with the property of transformation. One assay which may be utilized to determine transformation in colony formation of cells plated in soft agar (MacPherson et al., *Vir. 23*:291-294, 1964). Briefly, one property of normal non-transformed cells is anchorage dependent growth. Normal non-transformed cells will stop proliferating when they are in semi-solid agar support medium, whereas transformed cells will continue to proliferate and form colonies in soft agar.

HT1080 (ATCC CCL 121), a neoplastic cell line derived from human fibrosarcoma and known to cause tumors in 100% of nude mice, is used as the assay positive control. WI-38 (ATCC CCL 75), a diploid embryonic human lung cell line which is not tumorigenic in nude mice, is used as the assay negative control.

WI-38 cell lines are transduced with the vector construct as described in Example 6B. Duplicate samples of each of the transduced cell lines, HT1080, and WI-38, are cultured in agar. Briefly, a lower layer of 5.0 ml 0.8% Bactoagar (Difco, Detroit, Michigan) in DMEM 17% FBS is set on 60 mm tissue culture plates. This is overlaid with 2.0 ml 0.3% Bactoagar in the same medium with the cells suspended at a concentration of 5 x 10⁵ cells/ml. To reduce background clumps, each cell line is strained through a 70 µm nylon mesh before suspending in the agar solution. The plates are incubated at 37°C in a humidified atmosphere of 5% CO₂ for 14 days. Within 24 hours of plating, representative plates of each cell line are examined for cell clumps present at the time of plating. On day 13, the plates are stained with 1.0 ml INT viral stain (Sigma, St Louis, Missouri) and on day 14, they are scanned for colonies of 150 µm in diameter using a 1 mm eyepiece reticle.

Only colonies spanning 150 µm in any orientation are scored, because colonies of this size can be readily observed in all planes under the microscope and non-transformed cells rarely form colonies of this size. At the end of the assay, the plating efficiencies for each cell line are calculated as b/a x 100, where b equals the sum of colonies on all plates, and a equals the total number of cells plates. A non-transformed cell line is one which has a plating efficiency of lower than or equal to 0.001%. Therefore, a transformed cell line will have a plating efficiency of greater than 0.001% (Risser et al., *Virol. 59*:477-489, 1974).

### EXAMPLE 14

### ADMINISTRATION PROTOCOLS

### A. Mice

The mouse system is used to evaluate the induction of humoral and cell-mediated immune responses with direct administration of vector encoding HBV core or e antigen. Six- to eight-week-old female Balb/C, C57B16 or C3H mice are injected intramuscularly (i.m.) with 0.1 ml of reconstituted (with sterile deionized, distilled water) lyophilized HBV core or HBV e expressing retroviral vector. Two injections are given one week apart. Seven days after the second injection, the animals are sacrificed. The chromium release CTL assays are preferred essentially as described in Example 11A i.

### B. Chimpanzee Administration Protocol

The data generated in the mouse system from Example 14A is used to determine the protocol of administration of vector in chimpanzees chronically infected with hepatitis B virus. Based on the induction of HBV-specific CTLs in mice, the subjects in chimpanzee trials will receive three doses of vector encoding core or e antigen at 28 day intervals given in two successively escalating dosage groups. Control subjects will receive a placebo comprised of HBV-IT (V) formulation media. The dosage will be either 10⁶ or 10⁷ HBV-IT (V) cfu given in four 0.5 ml injections i.m. on each injection day. Blood samples will be drawn on days 4, 12, 24, 36, 52, 70 and 84 and months 6, 12, 18, 24, 30, and 36 in order to measure serum ALT levels, the presence of hepatitis B e antigen, the presence of antibodies directed against the hepatitis B e antigen and to assess safety and tolerability of the treatment. The hepatitis B e antigen is detected by Abbott HB e rDNA EIA kit as described in Example 10A. Antibodies to HB e antigen can be detected by Abbott HB e rDNA EIA kit and efficacy of the induction of CTLs against hepatitis B core or e antigen can be determined as in Example 11A iii. Based on the safety and efficacy results from the chimpanzee studies, the dosage and inoculation schedule will be determined for administration of the vector to subjects in human trials. There subjects are monitored for serum ALT levels, presence of hepatitis B e antigen and the presence of antibodies directed against the hepatitis B e antigen essentially as described above. Induction of human CTLs against hepatitis B core or e antigen is determined as in Example 11A.

## Claims

1. A recombinant retrovirus which carries a vector construct which directs the expression of at least one immunogenic portion of the hepatitis B antigen HBcAg for use in treating hepatitis B infections.

2. A recombinant retrovirus as claimed in claim 1 which directs the expression of at least one immunogenic portion of the hepatitis B antigen HBcAg and an immunomodulatory cofactor for use in treating hepatitis B infections.

3. A recombinant retrovirus as claimed in claim 1 which co-expresses at least one immunogenic portion of the hepatitis B antigen HBcAg and an immunomodulatory cofactor for use in treating hepatitis B infections.

4. A recombinant retrovirus which carries a vector construct which directs the co-expression of at least one immunogenic portion of the hepatitis B antigen HBcAg and of an immunomodulatory cofactor.

5. A pharmaceutical composition comprising a recombinant retrovirus according to any one of claims 1 to 4 in combination with a pharmaceutically acceptable carrier or diluent.

6. A kit for treating hepatitis B infections, which kit comprises a recombinant retrovirus as defined in claim 1 and an immunomodulatory co-factor.

## Patentansprüche

1. Rekombinantes Retrovirus, das ein Vektorkonstrukt trägt, welches die Expression mindestens eines immunogenen Teils des Hepatitis-B-Antigens HBcAg steuert, zur Verwendung bei der Behandlung von Hepatitis-B-Infektionen.

2. Rekombinantes Retrovirus nach Anspruch 1, das die Expression mindestens eines immunogenen Teils des Hepatitis-B-Antigens HBcAg steuert, und ein immunmodulatorischer Cofaktor zur Verwendung in der Behandlung von Hepatitis-B-Infektionen.

3. Rekombinantes Retrovirus nach Anspruch 1, das mindestens einen immunogenen Teil des Hepatitis-B-Antigens HBcAg und einen immunmodulatorischen Cofaktor coexprimiert, zur Verwendung bei der Behandlung von Hepatitis-B-Infektionen.

4. Rekombinantes Retrovirus, das ein Vektorkonstrukt trägt, das die Co-Expression mindestens eines immunogenen Teils des Hepatitis-B-Antigens HBcAg und eines immunmodulatorischen Cofaktors steuert.

5. Pharmazeutische Zusammensetzung, die ein rekombinantes Retrovirus nach einem der Ansprüche 1 bis 4 in Kombination mit einem pharmazeutisch annehmbaren Träger oder Verdünnungsmittel umfasst.

6. Kit zur Behandlung von Hepatitis-B-Infektionen; wobei der Kit ein rekombinantes Retrovirus nach Anspruch 1 und einen immunmodulatorischen Cofaktor umfasst.

## Revendications

1. Rétrovirus recombiné qui porte une construction de vecteur qui dirige l'expression d'au moins une partie immunogène de l'antigène d'hépatite B HBcAg, destiné à être utilisé dans le traitement d'infections par l'hépatite B.

2. Rétrovirus recombiné selon la revendication 1, qui dirige l'expression d'au moins une partie immunogène de l'antigène d'hépatite B HBcAg et d'un cofacteur immunomodulateur, destiné à être utilisé dans le traitement d'infections par l'hépatite B.

3. Rétrovirus recombiné selon la revendication 1, qui co-exprime au moins une partie immunogène de l'antigène d'hépatite B HBcAg et un cofacteur immunomodulateur destiné à être utilisé dans le traitement d'infections par l'hépatite B.

4. Rétrovirus recombiné qui porte une construction de vecteur qui dirige la co-expression d'au moins une partie immunogène de l'antigène d'hépatite B HBcAg et d'un cofacteur immunomodulateur.

5. Composition pharmaceutique comprenant un rétrovirus recombiné selon l'une quelconque des revendications 1 à 4 en combinaison avec un véhicule ou un diluant pharmaceutiquement acceptable.

6. Kit pour le traitement d'infections par l'hépatite B, ce kit comprenant un rétrovirus recombiné tel que défini dans la revendication 1 et un cofacteur immunomodulateur.
